# EUROPÄISCHE PATENTSCHRIFT

(11) **EP 1 401 851 B1**
(45) Veröffentlichungstag und Bekanntmachung des Hinweises auf die Patenterteilung: **02.07.2008**
(21) Anmeldenummer: 02754822.1
(22) Anmeldetag: 03.07.2002
(51) Int. Cl.: C07H 19/06, C07H 19/10, C07H 19/16, C07H 19/20, C07H 21/00

(54) **ZWEISTUFIGE SCHUTZGRUPPEN FÜR DIE SYNTHESE VON BIOPOLYMEREN**
TWO-STAGE PROTECTIVE GROUPS FOR THE SYNTHESIS OF BIOPOLYMERS
GROUPES PROTECTEURS SEPARES EN DEUX ETAPES POUR LA SYNTHESE DE BIOPOLYMERES

(30) Priorität: 03.07.2001 DE 10132025; 24.08.2001 US 314306 P
(43) Veröffentlichungstag der Anmeldung: 31.03.2004
(73) Patentinhaber: febit biotech GmbH, 69120 Heidelberg (DE)
(72) Erfinder: GÜIMIL, Ramon, 69126 Heidelberg (DE); SCHEFFLER, Matthias, 69493 Hirschberg/Leutershausen (DE); STÄHLER, Peer, F., 68167 Mannheim (DE); BEIJER, Barbro, 69226 Nussloch (DE)
(74) Vertreter: Weiss, Wolfgang
(86) Internationale Anmeldenummer: PCT/EP2002/007389
(87) Internationale Veröffentlichungsnummer: WO 2003/004510

(56) Entgegenhaltungen:
- US-A- 5 763 599
- E. HAPP, C. SCALFI HAPP: "New trityl-based protecting groups with a mild two-step removal" NUCLEOSIDES & NUCLEOTIDES, Bd. 7, 1988, Seiten 813-816, XP008011020

## Beschreibung

Die vorliegende Erfindung betrifft ein Verfahren zur Synthese von Biopolymeren durch schrittweisen Aufbau aus geschützten Synthesebausteinen, die zweistufige Schutzgruppen tragen. Die zweistufigen Schutzgruppen werden durch einen ersten Belichtungsschritt aktiviert und einen anschließenden chemischen Behandlungsschritt abgespalten.

Die Technologie der lichtgesteuerten Synthese von Biopolymeren unter Verwendung fotolabiler Schutzgruppen eröffnet die Möglichkeit, Biochips in situ durch Synthese aus Monomer- und Oligomerbausteinen zu erzeugen. Biochips haben für die Forschung und Diagnostik ganz erheblich an Bedeutung gewonnen, da sie eine schnelle und hochparallele Bearbeitung komplexer biologischer Fragestellungen erlauben. Hierzu werden jedoch Chips von höchster Qualität benötigt, so dass ein hohes Interesse an neuen effektiveren Synthesemethoden besteht.

Bei der lichtgesteuerten Synthese von Nukleinsäure-Chips finden fotolabile Nukleosid-Derivate Verwendung. Hierbei findet der Kettenaufbau der Nukleinsäure-Fragmente üblicherweise mittels Phosphoramidit-Synthonen statt. Die Bausteine tragen jeweils eine temporäre Fotoschutzgruppe, die durch Lichteinstrahlung entfernt werden kann. Das Syntheseprinzip sieht eine zyklische Abfolge von Kondensations- und Deprotektions-Schritten (durch Licht) vor. Die Effizienz, mit der eine solche lichtgesteuerte Synthese erfolgen kann, wird im Wesentlichen durch die verwendeten fotolabilen Schutzgruppen, insbesondere durch die Effizienz, mit der diese im Bestrahlungsschritt entfernt werden können, bestimmt. Bei den bislang zur lichtgesteuerten Synthese verwendeten Fotoschutzgruppen handelt es sich üblicherweise um die Schutzgruppen NVOC (S. P. A. Fodor et al., Science 251 (1991), 767 ff.), MeNPOC (A. C. Pease et al., Proc. Natl. Acad. Sci. 91 (1994), 5022 ff.), DMBOC (M. C. Pirrung, J. Chem. 60 (1995), 1116 ff.) und NPPOC (A. Hassan et al., Tetrahedron 53 (1997), 4247 ff.). Weitere bekannte fotolabile Schutzgruppen in der Nukleosid- bzw. Nukleotidchemie sind o-Nitrobenzyl-Gruppen und ihre Derivate (vgl. z.B. Pillai, Org. Photochem. 9 (1987), 225; Walker et al., J. Am. Chem.Soc. 110 (1988), 7170). Als weitere fotolabile Schutzgruppe wurde die 2-(o-Nitrophenyl)ethyl-Gruppe (Pfleiderer et al., In: "Biosphosphates an their Analogues - Synthesis, Structure, Metabolism and Activity", ELSEVIER Science Publishers B.V. Amsterdam (1987), 133 ff.) sowie Derivate davon (WO97/44345 und WO96/18634) vorgeschlagen.

Die gegenwärtig für die lichtgesteuerte Synthese von Nukleinsäuren verwendeten fotolabilen Schutzgruppen. (z.B. NVOC, MeNPOC, NPPOC) zeichnen sich im Allgemeinen durch einen vergleichsweise niedrigen Absorptionskoeffizienten bei der Wellenlänge der Lichteinstrahlung aus. Die Bestrahlung der fotolabilen Nukleosid-Derivate erfolgt üblicherweise mit Hg-Hochdrucklampen bei einer Wellenlänge von 365 nm. Der niedrige Absorptionskoeffizient der verwendeten fotolabilen Schutzgruppe bei dieser Wellenlänge hat zur Folge, dass nur ein sehr geringer Anteil des eingestrahlten Lichts zur Anregung der Moleküle verwertet werden kann. Desweiteren handelt es sich bei den verwendeten fotolabilen Schutzgruppen zumeist um farblose Derivate. Dies wiederum hat zur Folge, dass es während der Synthese nicht möglich ist, mit einfachen spektroskopischen Methoden zu detektieren, ob die fotolabile Schutzgruppe sich noch am Nukleosid-Derivat befindet oder bereits teilweise oder vollständig durch den Lichteintrag abstrahiert worden ist. Es lässt sich somit der Vorgang der Abstraktion nur schwer oder gar nicht verfolgen.

Muller et al. (Helvetica Chim. Acta 84 (2001), 3735-3741) beschreiben eine fotolabile Schutzgruppe, bestehend aus einer MeNPOC-Gruppe, an die ein fluoreszierendes Coumarinderivat über einen Aminolinker gekoppelt ist. Diese fotolabile Schutzgruppe wird zur Synthese von Oligonuklotiden auf DNA-Microarrays eingesetzt. Die Abspaltung der fotolabilen Schutzgruppe erfolgt in einem einzigen Schritt durch Bestrahlung.

Happ et al. (Nucleosides & Nucleotides 7 (1988), 813-816) offenbaren 4-(9-Fluorenylmethoxycarbonyl)oxy/amino-4',4"-dimethoxytrityl-Schutzgruppen, welche in zwei aufeinander folgenden chemischen Behandlungsschritten abgespalten werden und für die Synthese von Oligonukleotiden eingesetzt werden können.

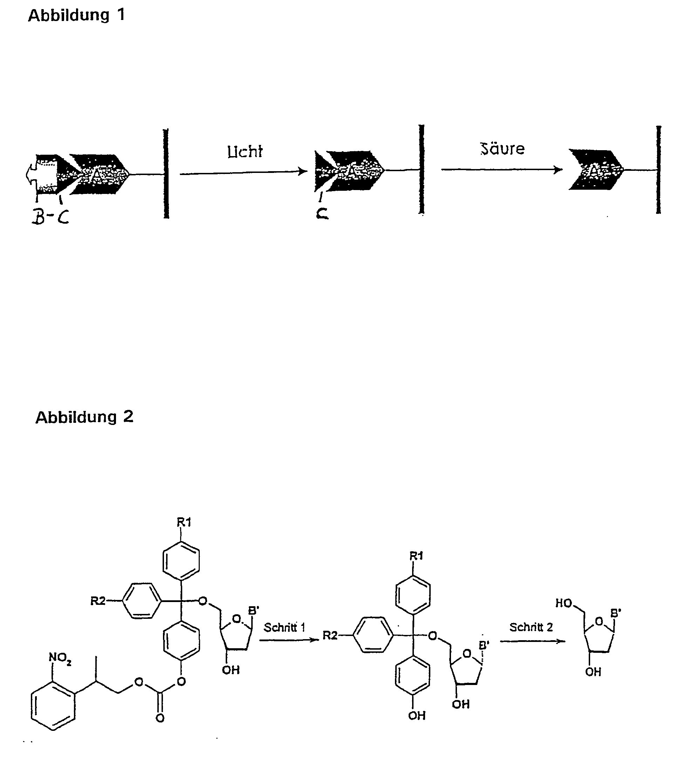

Gemäß vorliegender Erfindung wird eine neuartige Schutzgruppe bereitgestellt, bei der der Aktivierungsschritt lichtinduziert erfolgt und der eigentliche Entschützungsschritt am Reaktionszentrum durch chemische Mittel, z.B. säurekatalysiert, stattfindet (Abb. 1). Diese neuartige Schutzgruppe bzw. diese Schutzgruppe tragende Moleküle können für die Synthese von Biopolymeren eingesetzt werden.

Ein Gegenstand der Erfindung ist somit ein Verfahren zur Synthese von Biopolymeren durch schrittweisen Aufbau aus Synthesebausteinen, die Schutzgruppen tragen, wobei man mindestens einen Synthesebaustein verwendet, der eine zweistufige Schutzgruppe trägt, die durch einen Belichtungsschritt aktiviert und durch einen anschließenden chemischen Behandlungsschritt abgespalten wird. Der chemische Behandlungsschritt umfasst vorzugsweise eine Behandlung mit Base, eine Behandlung mit Säure, eine Oxidation, eine Reduktion oder/und eine enzymatische Reaktion. Besonders bevorzugt umfasst der chemische Behandlungsschritt eine Säurebehandlung.
In einer besonders bevorzugten Ausführungsform der Erfindung verwendet man als zweistufige Schutzgruppe eine derivatisierte Tritylgruppe. Tritylgruppen zeichnen sich durch ihre hervorragenden Abspaltbarkeit, insbesondere durch Behandlung mit Säure, aus. Die erfindungsgemäßen zweistufigen Tritylschutzgruppen sind hingegen nicht säurelabil, sondern werden erst nach Aktivierung und Abspaltung einer oder mehrerer fotolabiler Komponenten in eine säurelabile Form überführt. Besonders bevorzugt wird daher ein Synthesebaustein mit einer zweistufigen Schutzgruppe verwendet, der die allgemeine Formel I aufweist: 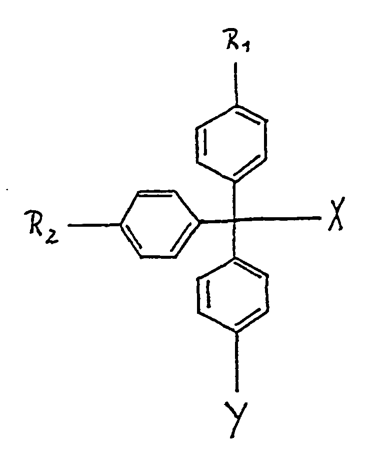 wobei R₁ und R₂ jeweils unabhängig ausgewählt sind aus Wasserstoff, OR₃, O(CH₂)ₙCOOR₃ und NHZ, R₃ eine C₁-C₈ Alkylgruppe, eine C₂-C₈-Alkenylgruppe, eine C₂-C₈-Alkinylgruppe oder/und eine C₆-C₂₀ Arylgruppe enthält, die gegebenenfalls einen oder mehrere Substituenten aufweisen kann, X den Synthesebaustein darstellt, Y jeweils unabhängig eine fotoaktivierbare Schutzgruppe ist, Z eine Aminoschutzgruppe ist, n eine ganze Zahl von 0 bis 4 ist und wobei gegebenenfalls R₁ oder/und R₂ durch Y ersetzt sein können.

Die Alkyl-, Alkenyl- und Alkinylgruppen können linear oder cyclisch, geradkettig oder verzweigt sein. Bevorzugte Bedeutungen für R₁ und R₂ sind Wasserstoff, O-Methyl, OCOO-Methyl oder eine geschützte Aminogruppe, z.B. eine mit einer geeigneten Carbonsäure in eine Amidfunktion überführte Aminogruppe.

Die Erfindung erfasst auch Verbindungen, die mehrere fotoaktivierbare Schutzgruppen tragen, insbesondere Verbindungen der Formel I, worin zumindest einer von R₁ oder R₂ durch eine fotoaktivierbare Schutzgruppe Y ersetzt ist.

Durch Variation der Reste R₁ und R₂ und Substitution eines oder beider Reste durch fotoaktivierbare Schutzgruppen lässt sich die Säurelabilität den gewünschten Anforderungen anpassen.

Die Erfindung umfasst auch Verbindungen, die eine oder mehrere fluoreszierende Gruppen tragen, z.B. Verbindungen, bei denen Y eine fluoreszierende Fotoschutzgruppe oder/und R₃ bzw. Z fluoreszierende Gruppen darstellen (R. Ramage, F.O. Wahl, Tetrahedron Lett., 34 (1993), 7133) oder Moleküle, bei denen die Fluoreszenz durch Substitution am Tritylgerüst (J.L. Fourrey et al., Tetrahedron Lett., 28 (1987), 5157) eingeführt wurde.

Diese fluoreszierenden Gruppen können, solange die Anregungs- und Emissionswellenlängen mit der lichtinduzierten Aktivierung nicht interferieren, zur Qualitätskontrolle von Biochips herangezogen werden. Dies kann beispielsweise in Biochip-Trägern gemäß WO 00/13018 geschehen.

Die fotoaktivierbare Gruppe Y der zweistufigen Schutzgruppe kann grundsätzlich aus beliebigen bekannten Fotoschutzgruppen ausgewählt werden, wie etwa Nitroveratryloxycarbonyl (NVOC), α-Methyl-6-nitropiperonyloxycarbonyl (MeNPOC), 3,5-Dimethoxybenzoincarbonat (DMBOC), 2-(o-Nitrophenyl)propyloxycarbonyl (NPPOC), o-Nitrobenzyl und Derivaten davon, 2-(o-Nitrophenyl)ethyl und Derivaten davon.

Falls mehrere fotoaktivierbare Gruppen Y vorhanden sind, können diese gleich oder verschieden sein.

Die fotoaktivierbare Komponente Y der zweitstufigen Schutzgruppe kann durch einen Belichtungsschritt abgespalten werden. Durch Abspaltung der Gruppe Y wird die Labilität der verbleibenden Schutzgruppe erhöht, so dass sie durch einen anschließenden chemischen Behandlungsschritt abgespalten werden kann, während eine zweistufige Schutzgruppe, welche die fotoaktivierbare Komponente Y noch enthält, unter derartigen Bedingungen im Wesentlichen gegenüber einer Abspaltung resistent ist.

Das erfindungsgemäße Verfahren wird zur Synthese von Biopolymeren eingesetzt, wobei das zu synthetisierende Biopolymer schrittweise aus mehreren Synthesebausteinen aufgebaut wird. Besonders bevorzugt wird das Verfahren zur Synthese von Nukleinsäuren, z.B. DNA oder RNA, eingesetzt. Es ist jedoch anzumerken, dass das Verfahren auch zur Synthese von anderen Biopolymeren, wie etwa Peptiden, Peptidnukleinsäuren (PNA) oder Sacchariden, geeignet ist. Der Synthesebaustein kann ein monomerer Baustein, z.B. ein Nukleosid-Derviat, aber auch ein oligomerer Baustein, z.B. ein Dimer oder Trimer, d.h. beispielsweise ein Di- oder Trinukleosid-Derivat, sein. Besonders bevorzugt ist der Synthesebaustein ein Phosphoramidit-Baustein. Es können jedoch auch andere Nukleotidsynthese-Bausteine, z. B. Phosphat- oder Phosphonat-Bausteine, verwendet werden. Weiterhin können als Synthesebausteine auch Linker- bzw. Spacer-Bausteine, z.B. als Phosphoramidite, eingesetzt werden. Besonders bevorzugte Linker bzw. Spacer als Träger zweistufiger Schutzgruppen sind in DE 100 41 539.3 beschrieben.

Die erfindungsgemäßen, eine zweistufige Schutzgruppe tragenden Synthesebausteine haben im Allgemeinen stärker lipophile Eigenschaften als die bislang die im Stand der Technik verwendeten Synthesebausteine. Durch diese Lipophilie erhöht sich die Solubilität der Synthesebausteine, insbesondere der Phosphoramidit-Synthone in organischen Lösungsmitteln. Die dadurch mögliche homogenere Reaktionsführung führt im Vergleich zu den reinen fotolabilen Phosphoramidit-Synthonen zu einer höheren Kopplungseffizienz. Durch die Abspaltung des farbigen Tritylkations der erfindungsgemäßen Fotoschutzgruppen, welches einen wesentlich höheren Absorptionskoeffizienten besitzt als die Abspaltungsprodukte bei anderen Fotoentschützungsprozessen, eröffnet sich weiterhin die Möglichkeit zur direkten online Prozesskontrolle. Dies führt zu einer Verbesserung bei der Qualitätskontrolle für Biochips.

Die Tritylgruppe der erfindungsgemäßen Fotoschutzgruppen ermöglicht außerdem die selektive Funktionalisierung der 5'-Hydroxyfunktion. Dies führt zu einer enormen Kostenreduzierung, da eine Trennung der 3'-5'-Isomere entfällt.

Besonders bevorzugt sind daher gemäß vorliegender Erfindung Phosphoramidit-Bausteine, die die zweistufige Schutzgruppe am 5'-O-Atom des Zuckers, insbesondere der Ribose oder der Deoxyribose, tragen.

Die Synthese der Biopolymeren kann auf übliche Weise durchgeführt werden, beispielsweise auf einer Festphase. Besonders bevorzugt werden mehrere Biopolymere, die eine unterschiedliche Sequenz von Synthesebausteinen tragen, auf einem einzigen Träger in Form eines Array in situ erzeugt.

Noch ein weiterer Gegenstand der Erfindung sind Verbindungen der allgemeinen Formel I 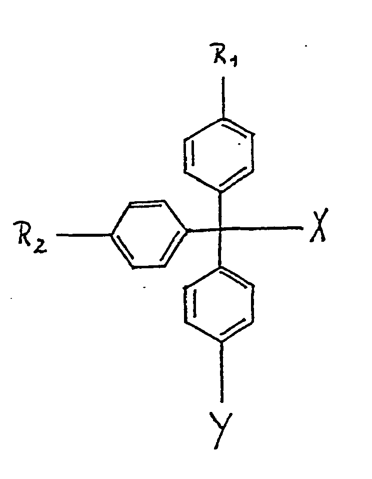 wobei R₁ und R₂ jeweils unabhängig ausgewählt sind aus Wasserstoff, OR₃, O(CH₂)ₙCOOR₃ und NHZ, R₃ eine C₁-C₈ Alkylgruppe oder/und eine C₆-C₂₀ Arylgruppe enthält, die gegebenenfalls Substituenten aufweisen kann, X einen Synthesebaustein zur Synthese von Biopolymeren oder eine Abgangsgruppe darstellt, Y jeweils unabhängig eine fotoaktivierbare Schutzgruppe ist, Z eine Aminoschutzgruppe ist, n eine ganze Zahl von 0 bis 4 ist und wobei gegebenenfalls R₁ oder/und R₂ durch Y ersetzt sein können.

Substituenten von Alkyl-, Alkenyl-, Alkinyl- bzw. Arylgruppen sind vorzugsweise ausgewählt aus Halogenen, z.B. F, Cl, Br oder I, OH, SH, -O-, -S-, - S(O)-, -S(O)₂-, NO₂, CN und NHZ, wobei Z eine Aminoschutzgruppe ist. Die Substituenten können an dem betreffenden Rest einfach oder mehrfach vorliegen. Arylgruppen können auch Ringsysteme mit Heteroatomen, wie etwa O, N oder/und S, umfassen. Alkyl-, Alkenyl- und Alkinylgruppen können in geradkettiger, verzweigtkettiger oder cyclischer Form vorliegen und gegebenenfalls mit einer C₆-C₂₀-Arylgruppe substituiert sein, wobei die Arylgruppe wiederum Substituenten wie zuvor angegeben enthalten kann. Substituenten von Arylgruppen werden z.B. ausgewählt aus -OH, Halogen, -C₁-C₄-Alkyl, -O-C₁-C₄-Alkyl, wobei die Alkylgruppen wie zuvor angegeben substituiert sein können.

Wenn X eine Abgangsgruppe ist, handelt es sich um eine Gruppe, die bei einer Reaktion der Verbindung (I) mit einer anderen Verbindung abgespalten werden kann. Vorzugsweise ist X eine durch Reaktion mit eine Nukleophil, gegebenenfalls in Gegenwart einer Hilfsbase, wie Pyridin, abspaltbare Abgangsgruppe. Bevorzugte Beispiele für X sind: Cl, Br, I, Tosylat, Mesylat, Trifluorsulfonat etc.

Die schematische Darstellung des erfindungsgemäßen Schutzgruppenkonzepts ist in **Abbildung 1** gezeigt. Der Synthesebaustein (A) trägt eine zweistufige Schutzgruppe (B-C). In einem ersten Belichtungsschritt wird der fotolabile Anteil (B) der Schutzgruppe abgespalten. Durch eine zweiten chemischen Behandlungsschritt, z.B. durch Zugabe von Säure, wird die chemisch labile Komponente (C) der Schutzgruppe abgespalten, so dass der Synthesebaustein (A) in aktiver Form vorliegt.

**Abbildung 2** zeigt eine Beispielsubstanz aus einer bevorzugten Klasse von erfindungsgemäßen zweistufigen Schutzgruppen. Sie basiert auf der säurelabilen Tritylgruppe, enthält jedoch in der p-Position eines Phenylrests eine fotolabile Komponente (hier die NPPOC-Gruppe), welche die Säureempfindlichkeit der Tritylgruppe verringert bzw. vollständig blockiert. Durch Belichtung und Abspaltung der fotolabilen Komponente wird die Schutzgruppe in eine säurelabile Form überführt und kann anschließend in Gegenwart von Säure unter Freisetzung des ungeschützten Synthesebausteins abgespalten werden.

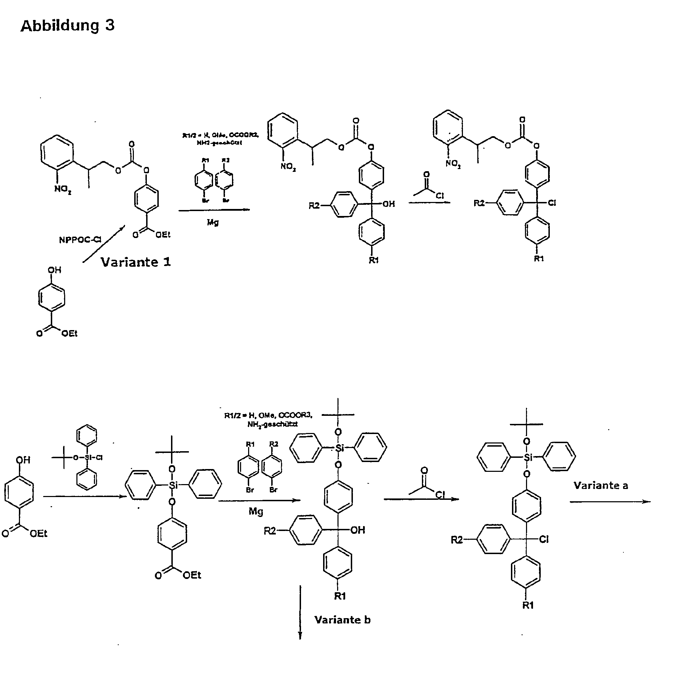

**Abbildung 3** zeigt zwei Varianten zur Synthese einer erfindungsgemäßen zweistufigen Schutzgruppe.

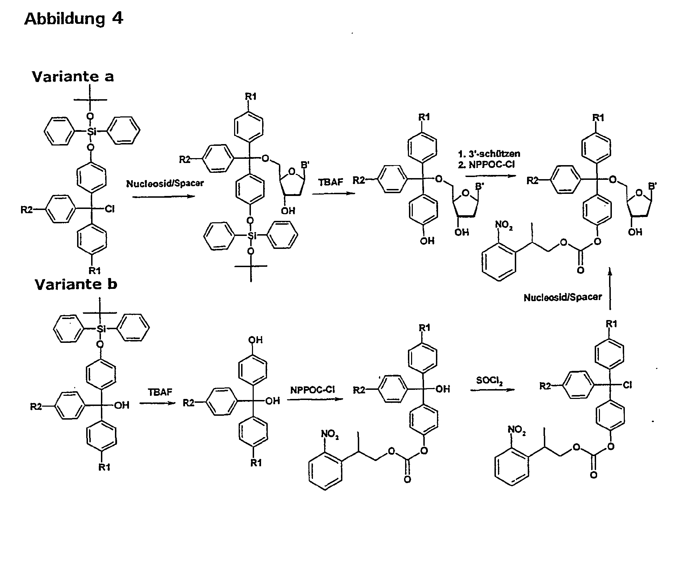

**Abbildung 4** zeigt zwei weitere Varianten zur Herstellung erfindungsmäßer Synthesebausteine mit zweistufigen Schutzgruppen.

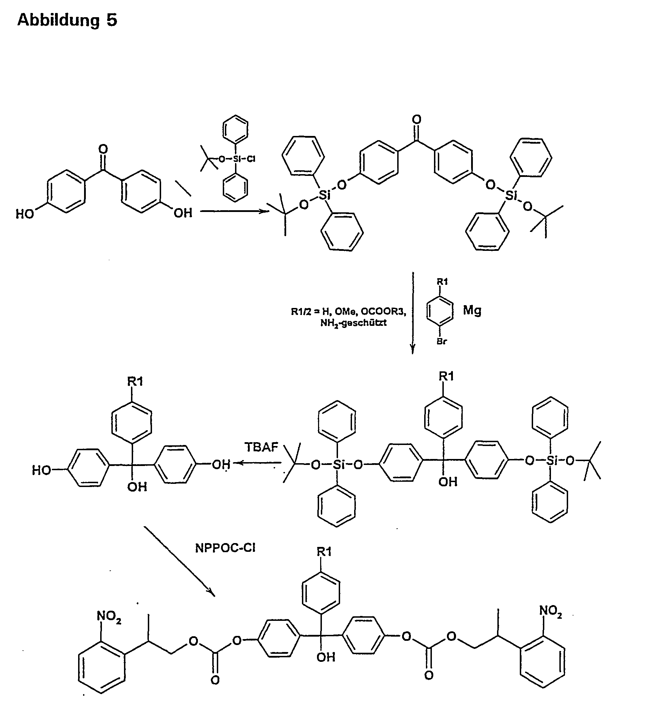

**Abbildung 5** zeigt eine dritte Variante der Synthese zweistufiger Schutzgruppen, wobei zwei der Phenylgruppen an der Tritylgruppe mit einer fotolabilen Gruppe substituiert sind.

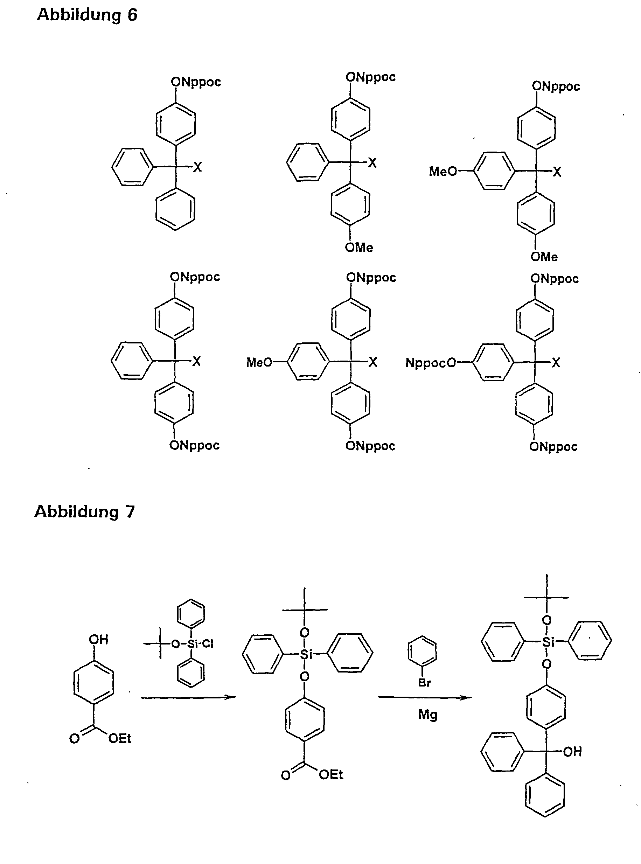

**Abbildung 6** zeigt konkrete Ausführungsformen für Carbonatderivate der zweistufigen Schutzgruppe und

**Abbildung 7** zeigt ein Verfahren zur Synthese der S1-Schutzgruppe. Ausgehend von 4-Hydroxybenzoesäureethylester und tert.-Butoxydiphenylchlorsilan in Anwesenheit von Imidazol, gelingt die quantitative Einführung der Silylgruppe. Der silylderivatisierte Ester wird mit dem Grignard-Reagenz des Brombenzols zum 4-(tert.- Butoxydiphenylsilyloxy)triphenylcarbinol (S1) umgesetzt. In analoger Weise gelangt man durch Verwendung des Bromanisols zum 4,4'-Dimethoxy-4"- (tert.-Butoxydiphenylsilyloxy)tritylcarbinol (S3, ohne Abb.).

**Abbildung 8A** zeigt die Synthese der S2-Schutzgruppe.
Das Methoxy-Silyloxy-Derivat ist, ausgehend von silylgeschütztem 4-Hydroxybenzophenon, welches in Analogie zum Hydroxybenzoesäureethylester in Gegenwart von Imidazol synthetisiert wird, durch Umsetzung mit dem Grignard-Reagenz des Bromanisols zum 4-tert.-Butoxydiphenylsilyloxytriphenylcarbinol (S2) zugänglich.

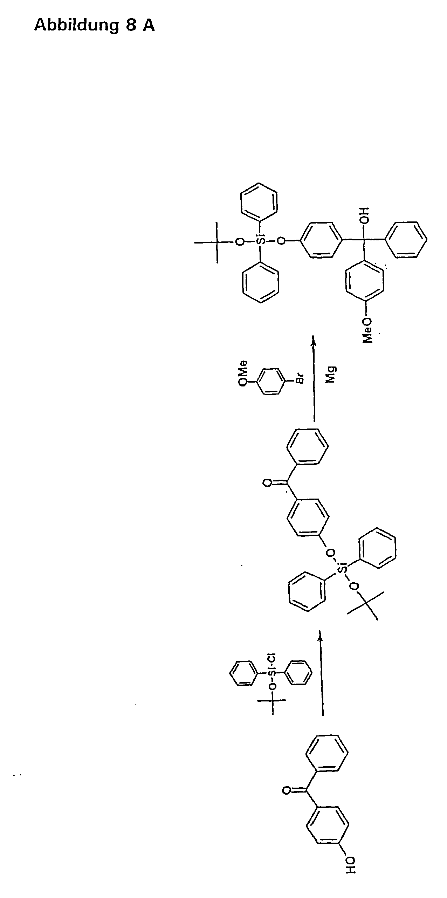

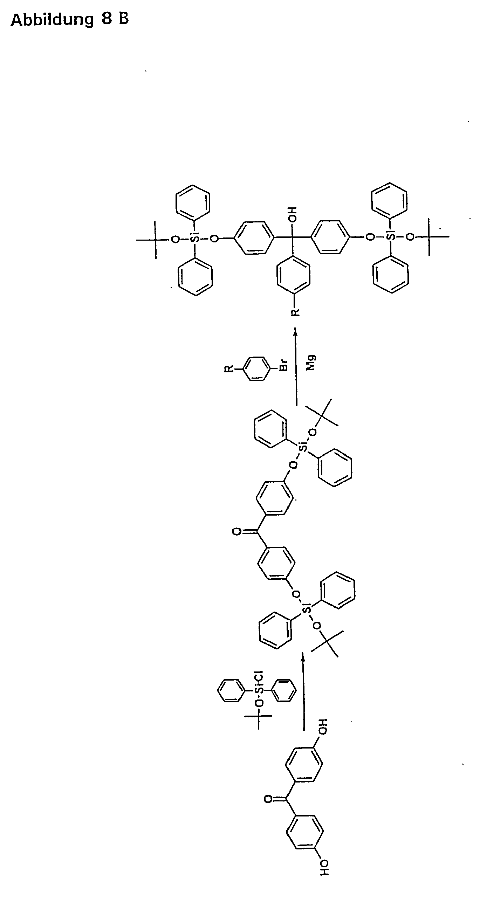

**Abbildung 8B** zeigt ein Syntheseschema für S4/5, R = H oder OMe.
Die Darstellung der Di-Silyloxy-Tritylcarbinolderivate (S4,5) erfolgt durch Umsetzung eines geschützten 4,4'-Dihydroxybenzophenons, welches entweder mit Brombenzol (R = H) oder mit Bromanisol (R = OMe) reagiert.

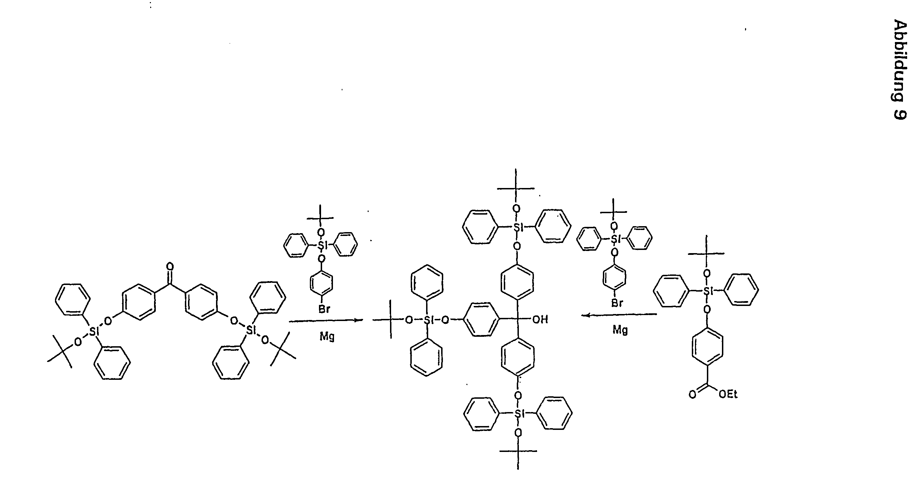

**Abbildung 9** zeigt die Synthese der S6-Schutzgruppe.
Die Tri-Silyloxy-Tritylcarbinolvariante (S6) wird entweder durch die Umsetzung von 4'-Silyloxy-Brombenzol mit 4'-Silylbenzoesäureethylester oder durch Umsetzung von 4,4'-Disilyloxybenzophenon und 4'-Silyloxybrombenzol hergestellt.

Die Abspaltung der tert.-Butoxydiphenylsilylgruppe aus den Silylgeschützten Verbindungen kannn durch einstündige Reaktion mit TBAF; Reaktionsabbruch durch Zugabe von Pyridin/Methanol/Wasser und Pyridinium-DOWEX erfolgen. Die Derivate können meist direkt mit Nppoc-Cl umgesetzt werden.

Bei der anschließenden Halogenierung der Carbinole zeigen sich unterschiedliche Eigenschaften, die durch die Variation der elektronenziehenden- bzw. elektronenschiebenden Substituenten in para-Stellung hervorgerufen werden.

Der Austausch des Alkohols durch ein Halogenid erfolgt nach dem Mechanismus der nukleophilen Substitution 1.Ordnung. Daraus ergibt sich, dass alle Prozesse über ein trigonalplanares Carbeniumion verlaufen. Entscheidend für diesen Mechanismus ist die Stabilität des Kations, dessen Struktur und die Möglichkeit zur Delokalisation der π-Etektronen. Zusätzlich wird es durch elektronenschiebende Substituenten stabilisiert und durch elektronenziehende Substituenten destabilisiert. In diesem Fall geht die Stabilität des Kations direkt mit der Säurebeständigkeit der Schutzgruppe einher. Je stabiler das Kation, desto säurelabiler sind die korrespondierenden Schutzgruppen.

In der Literatur sind eine Reihe von Standardverfahren beschrieben, die zur Halogenierung von Triphenylcarbinolderivaten herangezogen werden. Erfahrungen mit pyrensubstituierten Dimethoxytritylcarbinolen zeigen, dass sich extrem elektronenreiche Verbindungen innerhalb von Minuten mit Acetylchlorid in Cyclohexan quantitativ chlorieren lassen. Für ausgewählte Schutzgruppen mit elektronenziehenden Para-Substituenten zeigt es sich, dass mit refluxierendem SOCl₂ gute Umsätze erzielt werden.

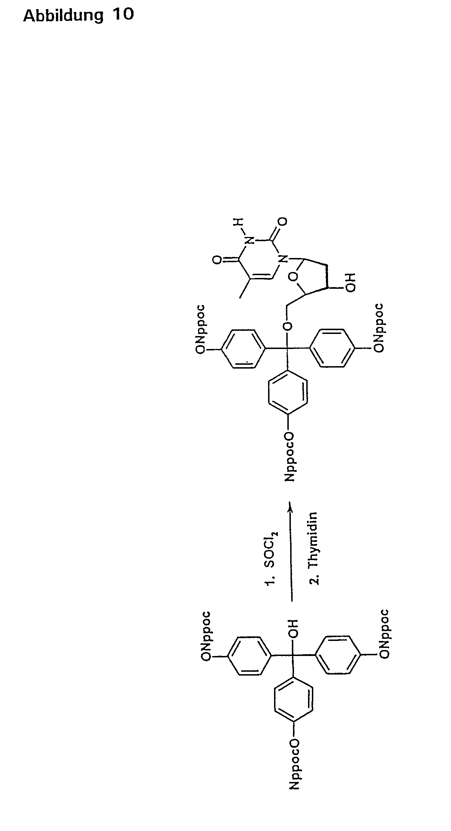

**Abbildung 10** zeigt die Herstellung von geschützten Thymidinderivaten.
Für Verbindungen mit mehreren Nppoc-Gruppen werden geringere Ausbeuten erhalten. Unter Verwendung von refluxierendem Acetylbromid bzw. SOBr₂ ist es gelungen, die Halogenderivate in Anwesenheit mehrerer Nppoc- Substituenten im analytischen Maßstab darzustellen und ohne weitere Aufarbeitung mit Thymidin umzusetzen.

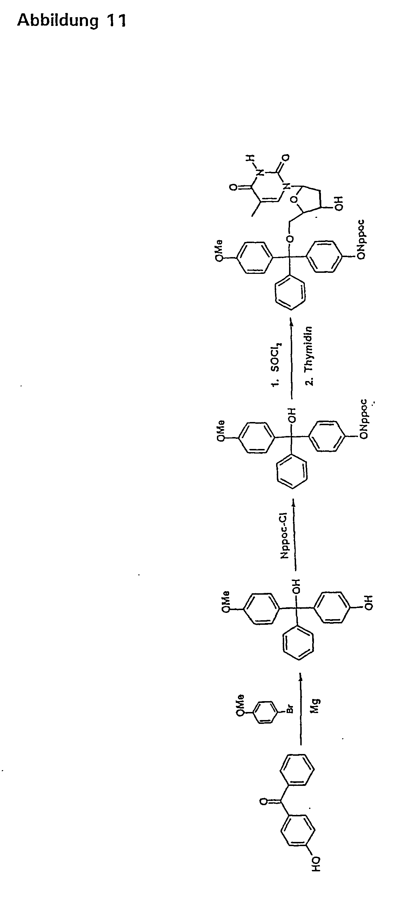

**Abbildung 11** zeigt ein weiteres Verfahren zur Synthese von Nukleotiden, die eine zweistufige Schutzgruppe tragen.

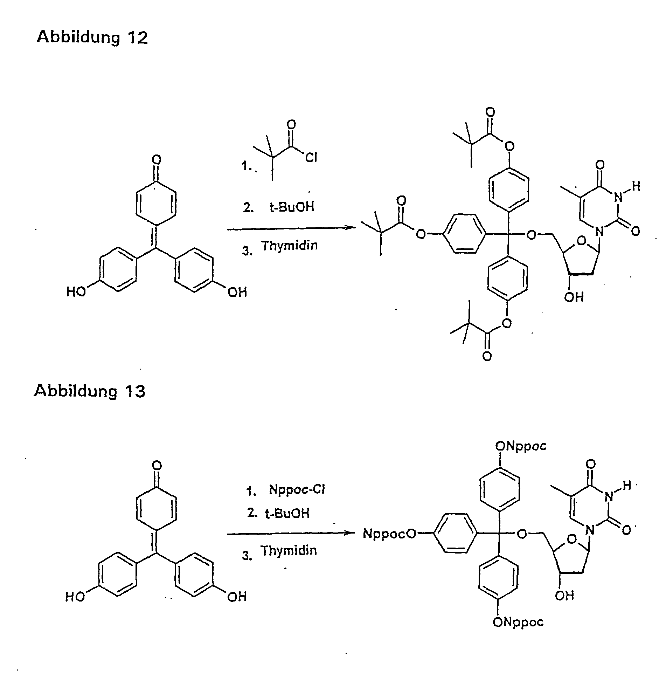

**Abbildung 12** und **Abbildung 13** zeigen Verfahren zur Herstellung von Nukleotiden, die eine zweistufige Schutzgruppe tragen, ausgehend von Rosolsäure.

Ausgehend von der Rosolsäure gelangt man zu dem Tri-Nppoc-Trityl-Thymidin in einer Eintopfreaktion. Bei diesem Ansatz kann auf die Verwendung teurer Schutzgruppen verzichtet werden. Als Modellverbindung für diesen Reaktionstyp diente das Tri-Pivaloyl-Tritylderivat.

Die Reaktion läuft in einem polar aprotischen Lösungsmittel bei 50 °C innerhalb von Minuten über eine Zwischenstufe, die dann mit den entsprechenden Nukleosiden zu den tandemgeschützten Derivaten weiterreagiert. Die Darstellung erfolgt im präparativen Maßstab.

### Beispiele

### Beispiel 1: Synthese von Tandemschutzgruppen

### 1.1 4-(tert.Butoxydiphenylsilyloxy)-triphenylcarbinol (S1)

In einem ausgeheizten Kolben wurden 0,61 g (25,00 mmol, 2,1 eq) Magnesium mit 20 ml THF unter Argon suspendiert. 6,28 g (40,00 mmol, 3,4 eq) Brombenzol wurden mit einem Tropftrichter langsam unter konstantem Sieden zugetropft. Nach Beendigung der Zugabe wurde mit einem Ölbad auf 85 °C erhitzt und 15 min lang nachgerührt. Das entstandene Magnesiumbromid wurde auf 70 ° C abkühlen gelassen und 5,00 g (11,89 mmol, 1 eq) 4-tert. Butoxydiphenylsilyloxybenzoesäureethylester gelöst in 20 ml THF zugetropft und für weitere 1,5 h bei 85 ° C erwärmt. Anschließend wurde der Ansatz erkalten gelassen und mit gesättigter Amoniumchlorid-Lösung abgebrochen. Zum Extrahieren wurden 500 ml Ethylacetat zugegeben und 2x mit Amoniumchlorid-Lösung (sat.) und 1 x mit Natriumchlorid-Lösung (sat.) extrahiert. Die organische Phase wurde mit Natriumsulfat getrocknet und eingedampft. Der Rückstand wurde säulenchromatographisch an Kieselgel 40-63 µm mit dem Eluent Ethylacetat/n-Hexan+1% TEA (1:7) gereinigt. Man erhält das 4-(tert. Butoxydiphenylsilyloxy)-triphenylcarbinol als weißen pulvrigen Feststoff.

Ausbeute: 5,75 g ≈ 91 % d. Th.

R_{f}-Wert _{(Ethylacetat / n-Hexan 1:7 +1% TEA)}: 0,19

### 1.2 4,4'-Dimethoxy-4"-(tert.butoxydiphenylsilyloxy)-triphenylcarbinol (S3)

In einem ausgeheizten Kolben wurden 2,78 g (114,14 mmol, 2,4 eq) Magnesium mit 80 ml THF unter Argon suspendiert. 26,68 g (142,67 mmol, 3,0 eq) Bromanisol wurden mit einem Tropftrichter langsam unter konstantem Sieden zugetropft. Nach Beendigung der Zugabe wurde mit einem Ölbad auf 85 °C erhitzt und 15 min lang nachgerührt. Das entstandene Magnesiumbromid wurde auf 70 °C abkühlen gelassen und 20,00 g (47,56 mmol, 1 eq) 4-tert. Butoxydiphenylsilyloxybenzoesäureethylester gelöst in 100 ml THF zugetropft und für weitere 2 h bei 85 °C erwärmt. Anschließend wurde der Ansatz erkalten gelassen und mit gesättigter Amoniumchlorid-Lösung abgebrochen. Zum Extrahieren wurden 500 ml Ethylacetat zugegeben und 2x mit Amoniumchlorid-Lösung (sat.) und 1x mit Natriumchlorid-Lösung (sat.) extrahiert. Die organische Phase wurde mit Natriumsulfat getrocknet und eingedampft. Der Rückstand wurde säulenchromatographisch nach dem Stufengradientenverfahren an Kieselgel 40-63 µm mit dem Eluent Ethylacetat/n-Hexan + 1 % TEA (1:5 und 1:3) gereinigt Man erhält das 4,4'-Dimethoxy-4''-(tert.butoxydiphenylsilyloxy)-triphenylcarbinol als weißen pulvrigen Feststoff.

Ausbeute: 25,65 g ≈ 97 % d. Th.

R_{f}-Wert _{(Ethylacetat /n-Hexan 1:3 +1 % TEA)}: 0,59

### 1.3 4-Methoxy-4'-(tert.butoxydiphenylsilyloxy)-triphenylcarbinol (S2)

In einem ausgeheizten Kolben wurden 0,64 g (26,51 mmol, 1,2 eq) Magnesium mit 20 ml THF unter Argon suspendiert. 6,20 g (33,14 mmol, 1,5 eq) Bromanisol wurde mit einem Tropftrichter langsam unter konstantem Sieden zugetropft. Nach Beendigung der Zugabe wurde mit einem Ölbad auf 85 °C erhitzt und 15 min lang nachgerührt. Das entstandene Magnesiumbromid wurde auf 70 °C abkühlen gelassen und 10,00 g (22,09 mmol, 1 eq) 4-tert.Butoxydiphenylsilyloxybenzophenon gelöst in 50 ml THF zugetropft und für weitere 2 h bei 70 ° C erwärmt. Anschließend wurde der Ansatz erkalten gelassen und mit gesättigter Amoniumchlorid-Lösung abgebrochen. Zum Extrahieren wurden 250 ml Ethylacetat zugegeben und 2x mit Amoniumchlorid-Lösung (sat.) und 1 x mit Natriumchlorid-Lösung (sat.) extrahiert. Die organische Phase wurde mit Natriumsulfat getrocknet und eingedampft. Auf eine Aufarbeitung wurde verzichtet. Man erhält das 4-Methoxy-4'-(tert. Butoxydiphenylsilyloxy)-triphenylcarbinol.

Ausbeute: quantitative Umsetzung

R_{f}-Wert _{(Ethylacetat /n-Hexan 1:3 + 1 % TEA)}: 0,58

### 1.4 4,4'-Di-(tert.butoxydiphenylsilyloxy)-triphenylcarbinol (S4)

In einem ausgeheizten Kolben wurden 0,20 g (8,28 mmol, 1,2 eq) Magnesium mit 20 ml THF unter Argon suspendiert. 1,63 g (10,35 mmol,1,5 eq) Brombenzol wurde mit einem Tropftrichter langsam unter konstantem Sieden zugetropft. Nach Beendigung der Zugabe wurde mit einem Ölbad auf 85 °C erhitzt und 15 min lang nachgerührt. Das entstandene Magnesiumbromid wurde auf 70 °C abkühlen gelassen und 5,00 g (6,90 mmol, 1 eq) 4,4'-di-tert.Butoxydiphenylsilyloxybenzophenon gelöst in 50 ml THF zugetropft und für weitere 2 h bei 70 °C erwärmt. Anschließend wurde der Ansatz erkalten gelassen und mit gesättigter Amoniumchlorid-Lösung abgebrochen. Zum Extrahieren wurden 250 ml Ethylacetat zugegeben und 2x mit Amoniumchlorid-Lösung (sat.) und 1x mit Natriumchlorid-Lösung (sat.) extrahiert. Die organische Phase wurde mit Natriumsulfat getrocknet und eingedampft. Der Rückstand wurde säulenchromatographisch an Kieselgel 40-63 µm mit dem Eluent Ethylacetat/n-Hexan + 1 % TEA (1:7) gereinigt. Man erhält das 4,4'-Di-(tert. butoxy.diphenylsilyloxy)-triphenylcarbinol als weißen pulvrigen Feststoff.

Ausbeute: 3,00 g ≈ 54 % d. Th.

R_{f}-Wert _{(Ethylacetat /n-Hexan 1:7 +1 % TEA)}: 0,27.

### 1.5 4,4'-Di-(tert.butoxydiphenylsilyloxy)-4"-methoxy-triphenylcarbinol (S5)

In einem ausgeheizten Kolben wurden 0,20 g (8,28 mmol, 1,2 eq) Magnesium mit 20 ml THF unter Argon suspendiert. 1,94 g (10,35 mmol, 1,5 eq) Bromanisol wurde mit einem Tropftrichter langsam unter konstantem Sieden zugetropft. Nach Beendigung der Zugabe wurde mit einem Ölbad auf 85 °C erhitzt und 15 min lang nachgerührt. Das entstandene Magnesiumbromid wurde auf 70 °C abkühlen gelassen und 5,00 g (6,90 mmol, 1 eq) 4,4'-Di-tert.butoxydiphenylsilyloxybenzophenon gelöst in 50 ml THF zugetropft und für weitere 2 h bei 70 °C erwärmt. Anschließend wurde der Ansatz erkalten gelassen und mit gesättigter Amoniumchlorid-Lösung abgebrochen. Zum Extrahieren wurden 250 ml Ethylacetat zugegeben und 2x mit Amoniumchlorid-Lösung (sat.) und 1x mit Natriumchlorid-Lösung (sat.) extrahiert. Die organische Phase wurde mit Natriumsulfat getrocknet und eingedampft. Der Rückstand wurde säulenchromatographisch an Kieselgel 40-63 µm mit dem Eluent Ethylacetat/n-Hexan+1 % TEA (1:5) gereinigt. Man erhält das 4,4'-Di-(tert.butoxydiphenylsilyloxy)-4"-methoxy-triphenylcarbinol als weißen pulvrigen Feststoff.

Ausbeute: 3,00 g ≈ 52 % d. Th.

R_{f}-Wert _{(Ethylacetat / n-Hexan 1:5 +1 % TEA)}: 0,26

### 1.6 4,4',4"-Tri-(tert.butoxydiphenylsi)yloxy)-triphenylcarbinol (S6)

In einem ausgeheizten Kolben wurden 0,41 g (16,87 mmol,1,2 eq) Magnesium mit 20 ml THF unter Argon suspendiert. 18,90 g (20,86 mmol, 1,5 eq) 4-tert.Butoxydiphenylsilyloxybrombenzol wurde mit einem Tropftrichter langsam unter konstantem Sieden zugetropft. Nach Beendigung der Zugabe wurde mit einem Ölbad auf 85 °C erhitzt und 15 Minuten lang nachgerührt. Das entstandene Magnesiumbromid wurde auf 70 °C abkühlen gelassen und 1 eq 10,10 g (13,97 mmol) 4,4'-di-tert. Butoxydiphenylsilyloxybenzophenon gelöst in 50 ml THF zugetropft und für weitere 2 h bei 70 °C erwärmt. Anschließend wurde der Ansatz erkalten gelassen und mit gesättigter Amoniumchlorid-Lösung abgebrochen. Zum Extrahieren wurden 250 ml Ethylacetat zugegeben und 2x mit Amoniumchlorid-Lösung (sat.) und 1x mit Natriumchlorid-Lösung (sat.) extrahiert. Die organische Phase wurde mit Natriumsulfat getrocknet und eingedampft. Der Rückstand wurde säulenchromatographisch an Kieselgel 40-63 µm mit dem Eluent Ethylacetat/n-Hexan + 1 % TEA (1:10) gereinigt. Manerhältdas4,4',4"-Tri-(tert.Butoxydiphenylsilyloxy)-triphenylcarbinol als weißen pulvrigen Feststoff.

Ausbeute: 10,20 g ≈ 68 % d. Th.

R_{f}-Wert _{(Ethylacetat / n-Hexan 1:10 +1 % TEA)}: 0,23

### 1.7 4-(2-[2-Nitrophenyl]-propyloxycarbonyloxy)-triphenylcarbinol

Zu 2,87 g (5,41 mmol, 1 eq) 4-tert.Butoxydiphenylsilyloxytriphenylearbinol gelöst in 20 ml THF wurden 6,00 ml (6,00 mmol, 1,1 eq) einer 1 M-TBAF-Lösung getropft und 1 h bei Raumtemperatur (RT) gerührt. Um die Reaktion abzubrechen wurden 5 ml Pyridin/MeOH/Wasser (3:1:1) und Pyridinium-Dowex zugegeben. Nach 30 min wurde abfiltriert und mit Pyridin gespült. Anschließend wurde die Lösung mehrmals mit Pyridin coevaporiert. Zu dem in 60 ml Pyridin/Methylenchlorid (2:1) gelösten Ansatz wurden 1,97 g (8,12 mmol, 1,5 eq) Nppoc-Cl gelöst in 10 ml Methylenchlorid getropft und 16 h bei RT gerührt. Danach wurde eingeengt und in Methylenchlorid aufgenommen, 2x mit Natriumhydrogencarbonat-Lösung (1 N) und 1x mit Natriumchlorid-Lösung (sat.) extrahiert. Die organische Phase wurde getrocknet und eingedampft. Der Rückstand wurde säulenchromatographisch an Kieselgel 40-63 µm mit dem Eluent Ethylacetat/n-Hexan+1 % TEA (1:2) gereinigt. Man erhält das 4-Nppoc-triphenylcarbinol als weißen festen Schaum.

Ausbeute: 2,16 g ≈ 83 % d. Th.

R_{f}-Wert _{(Ethylacetat / n-Hexan 1:1 +1 % TEA)}: 0,68

### 1.8 4-Methoxy-4'-(2-[2-nitrophenyl]-propyloxycarbonyloxy)-triphenylcarbinol (S2)

Zu 8,00 g (14,27 mmol, 1 eq,) 4-Methoxy-4'-tert.Butoxydiphenylsilyloxytriphenylcarbinol gelöst in 50 ml THF wurden 15,70 ml (15,70 mmol, 1,1 eq) 1M-TBAF-Lösung getropft und für 1 h bei RT gerührt. Um die Reaktion abzubrechen wurden 20 ml Pyridin/MeOH/Wasser (3:1:1) und Pyridinium-Dowex zugegeben. Nach 30 min wurde abfiltriert und mit Pyridin gespült. Anschließend wurde die Lösung mehrmals mit Pyridin coevaporiert. Zu dem in 60 ml Pyridin/Methylenchlorid (2:1) gelösten Ansatz wurden 6,50 g (26,75 mmol, 1,9 eq) Nppoc-Cl gelöst in 15 ml Methylenchlorid getropft und 16 h bei RT gerührt. Danach wurde eingeengt und in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch nach den Stufengarientverfahren an Kieselgel 40-63 µm mit dem Eluent Ethylacetat/n-Hexan + 1 % TEA (1:3 und 1:2) gereinigt. Man erhält das 4-Methoxy-4'-Nppoc-triphenylcarbinol als weißen festen Schaum.

Ausbeute: 5,5 g ≈ 75 % d. Th.

R_{f}-Wert _{(Ethylacetat / n-Hexan 1:1 + 1 % TEA)}: 0,54
¹³C-NMR: (125.75 MHz, CDCl₃, TMS): δ=17.70 (CH3-nppoc), 33.19 (CH-nppoc), 55.16 (CH3-O), 72.09 (CH2-nppoc), 81.31 (C-trityl), 113.23 (C-3,5-methoxyphenyl(trityl)), 120.04 (C-3,5-nppocphenyl(trityl)), 124.36 (C-3-phenyl-nppoc), 126.83 (C-4-phenyl(trityl)), 127.71 (C-2,6-phenyl(trityl)), 127.91 (C-6-phenyl-nppoc), 128.28 (C-4-phenyl-nppoc), 129.03 (C-3,5-phenyl(trityl)), 129.11 (C-2,6-nppocphenyl(trityl)), 129.35 (C-2,6-methoxyphenyl(trityl)), 132.76 (C-5-phenyl-nppoc), 136.63 (C-1-phenyl-nppoc), 138.88 (C-1-methoxyphenyl(trityl)), 144.87 (C-1-nppocphenyl(trityl)), 146.75 (C-1-phenyl(trityl)), 149.93 (C-2-phenyl-nppoc), 150.18 (O(CO)O), 153.35 (C-4-nppocphenyl(trityl)), 158.71 ppm (C-4-methoxyphenyl(trityl)).

### 1.9 4,4'-Dimethoxy-4"-(2-[2-nitrophenyl]-propyloxycarbonyloxy)-triphenylcarbinol (S3)

Zu 4,00 g (7,23 mmol, 1 eq) 4,4'-Dimethoxy-4"-tert.Butoxydiphenylsilyloxytriphenylcarbinol gelöst in 20 ml THF wurden 8,00 ml (8,00 mmol, 1,1 eq) 1M-TBAF-Lösung getropft und 1 h bei RT nachgerührt. Um die Reaktion abzubrechen wurden 10 ml Pyridin/MeOH/Wasser (3:1:1) und Pyridinium-Dowex zugegeben. Nach 30 min wurde abfiltriert mit Pyridin gespült und eingeengt. Der Rückstand wurde säulenchromatographisch nach dem Stufengradientverfahren über Kieselgel 40-63 µm, Eluent Ethylacetat/n-Hexan + 1 % TEA (1:3 und 1: 1), gereinigt. Zu 0,40 g (1,25 mmol) des gewonnenen 4,4'-Dimethoxy-4"-hydroxytriphenylcarbinols, gelöst in 30 ml Pyridin/Methylenchlorid (2: 1), wurden 0,40 g (1,70 mmol, 1,4 eq) Nppoc-Cl, gelöst in 5 ml Methylenchlorid, zugetropft. Der Ansatz wurde 16 h bei RT gerührt. Danach wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch an Kieselgel 40-63 µm mit dem Eluent Ethylacetat/n-Hexan+1 % TEA (1:3) gereinigt. Man erhält das 4,4'-Dimethoxy-4"-Nppoc-triphenylcarbinol als weißen festen Schaum.

Ausbeute: 0,57 g ≈ 86 % d. Th.

R_{f}-Wert _{(Ethylacetat / n-Hexan 1:1 + 1 % TEA)}: 0,59

### 1.10 4,4'-Di-(2-[2-nitrophenyl]-propyloxycarbonyloxy)-triphenylcarbinol (S4)

Zu 2,00 g (2,40 mmol, 1 eq) 4,4'-Di-tert.Butoxydiphenylsilyloxytriphenylcarbinol, gelöst in 30 ml THF, wurden 5,28 ml (5,28 mmol,2,2 eq) 1 M-TBAF-Lösung zugetropft und 1 h bei RT gerührt. Um die Reaktion abzubrechen wurden 20 ml Pyridin/MeOH/Wasser (3:1:1) und Pyridinium-Dowex zugegeben. Nach 30 min wurde abfiltriert und mit Pyridin gespült. Anschließend wurde die Lösung mehrmals mit Pyridin coevaporiert. Zu dem in 60 ml Pyridin/Methylenchlorid (2:1) gelösten Ansatz wurde 2,19 g (9,00 mmol, 3,7 eq) Nppoc-Cl, gelöst in 10 ml Methylenchlorid, zugetropft und 16 h bei RT gerührt. Danach wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch an Kieselgel 40-63 µm mit dem Eluent Ethylacetat/n-Hexan + 1 % TEA (1:1) gereinigt. Man erhält das 4,4'-Di-Nppoc-triphenylcarbinol als weißen festen Schaum.

Ausbeute: 0,75g ≈ 40,9 % d. Th.

R_{f}-Wert _{(Ethylacetat / n-Hexan 1:1 + 1 % TEA)}: 0,55

### 1.11 4-Methoxy-4',4"-di-(2-[2-nitrophenyl]-propyloxycarbonyloxy)-triphenylcarbinol (S5)

Zu 7,85 g (9,44 mmol, 1 eq) 4-Methoxy-4',4"-di-tert.butoxydiphenylsilyloxytriphenylcarbinol, gelöst in 50 ml THF, wurden 20,77 ml (20,77 mmol, 2,2 eq) 1 M-TBAF-Lösung zugetropft und 1 h bei RT gerührt. Um die Reaktion abzubrechen wurden 20 ml Pyridin/MeOH/Wasser (3:1:1) und Pyridinium-Dowex zugegeben. Nach 30 min wurde abfiltriert und mit Pyridin gespült. Anschließend wurde die Lösung mehrmals mit Pyridin coevaporiert. Zu dem in 60 ml Pyridin/Methylenchlorid (2:1) gelösten Ansatz wurden 6,10 g (25,03 mmol 2,6 eq) Nppoc-Cl, gelöst in 10 ml Methylenchlorid, zugetropft und 16 h bei RT gerührt. Danach wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch nach dem Stufengradientenverfahren an Kieselgel 40-63 µm mit dem Eluent Ethylacetat/n-Hexan + 1 % TEA (1:2, 2:3 bis 1:1) gereinigt. Man erhält das 4-Methoxy-4',4"-di-Nppoc-triphenylcarbinol als weißen festen Schaum.

Ausbeute: 3,0 g ≈ 43 % d. Th.

R_{f}-Wert _{(Ethylacetat / n-Hexan 1:1 + 1 % TEA)}: 0,65

### 1.12 4,4',4"-Tri-(2-[2-nitrophenyl]-propyloxycarbonyloxy)-triphenylcarbinol (S6)

Zu 10,20 g (9,53 mmol,1 eq) 4,4',4"-Tri-tert.Butoxydiphenylsilyloxytriphenylcarbinol, gelöst in 200 ml THF, wurden 37,45 ml (31,45 mmol, 3,3 eq) 1M-TBAF-Lösung zugetropft und 1 h bei RT gerührt. Um die Reaktion abzubrechen wurden 20 ml Pyridin/MeOH/Wasser (3:1:1) und Pyridinium-Dowex zugegeben. Nach 30 min wurde abfiltriert und mit Pyridin gespült. Anschließend wurde die Lösung mehrmals mit Pyridin coevaporiert. Zu dem in 120 ml Pyridin/Methylenchlorid (2:1) gelösten Ansatz wurde 9,29 g (38,12 mmol, 4,0 eq) Nppoc-Cl, gelöst in 20 ml Methylenchlorid, zugetropft und 16 h bei RT gerührt. Danach wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch nach dem Stufengradientenverfahren an Kieselgel 40-63 µm mit dem Eluent Ethylacetat/n-Hexan + 1 % TEA (1:2, bis 1: 1) gereinigt. Man erhält das 4,4',4"-Tri-Nppoc-triphenylcarbinol als weißen festen Schaum.

Ausbeute: 3,9 g ≈ 44 % d. Th.

R_{f}-Wert _{(Ethylacetat / n-Hexan 1:1 + 1 % TEA)}: 0,49
¹³C-NMR: (125.75 MHz, CDCl₃, TMS): δ=17.68 (CH3-nppoc), 33.17 (CH-nppoc), 72.14 (CH2-nppoc), 80.89 (C-trityl), 120.31 (C-3,3', 3",5,5',5"-trityl), 124.35 (C-3-phenyl-nppoc), 127.83 (C-6-phenyl-nppoc), 128.27 (C-4-phenyl-nppoc), 128.99 (C-2,2',2",6,6',6"-trityl), 132.77 (C-5-phenyl-nppoc), 136.62 (C-1-phenyl-nppoc), 144.09 (C-1,1', 1"-trityl), 150.14 (O(CO)O), 150.17 (C-2-phenyl-nppoc), 153.36 ppm (C-4,4',4"-trityl).
MS (TOF, ES+): 952,29 [M + Na], 968,25 [M + K].
MS (TOF, ES-): 928,42 [M-H], 694,37 [M-Cl].
C₄₉H₄₃N₃O₁₆:929,90

### 1.13 Halogenierung und Veretherung zu den 5'-O-Nppoc-triphenylmethyl-Thymidin Derivaten

1 eq des Triphenylcarbinol-Derivates wurden mit 100 eq Thionylchlorid für 3 h bei 90 ° C erhitzt. Anschließend wurde Methylenchlorid zugegeben und weitere 2 h erhitzt. Danach wurde mit Cyclohexan 3x eingeengt. Das entstandene Triphenylmethylchlorid-Derivat wurde in Methylenchlorid gelöst und unter Schutzgas 2 eq Thymidin, gelöst in Pyridin und Methylenchlorid, zugetropft. Nach 16 h Rühren bei RT wurde die Reaktion durch Zugabe von Ethanol abgebrochen. Danach wurde eingeengt, in Methylenchlorid gelöst und 2x mit Natriumhydrogencarbonat-Lösung (1 N) und 1x mit gesättigter Natriumchlorid-Lösung extrahiert.Die organische Phase wurde getrocknet und eingedampft. Der Rückstand wurde säulenchromatographisch gereinigt an Kieselgel 40-63 µm. Laufmittel und Ausbeuten sind der folgenden Tabelle 1 zu entnehmen:

**Tabelle 1:**

| Substanz | Kochzeit SOCl₂ | R_{f}-Wert: Eluent + 1 % TEA | Ausbeute |
|---|---|---|---|
| 5'-O-(4-Nppoc-trityl)-thymidin | 2+3 h | (CH₂Cl₂ + 5 % MeOH) 0,26 | 95 % |
| 5'-O-(4-Methoxy-4'-Nppoc-trityl)-thymidin | 3+2 h | (EE/Hex2:1) 0,18 | 50 % |
| 5'-O-(4,4'-Dimethoxy-4"Nppoc-trity)I-thymidin | 2+3 h | (CH₂Cl₂ +5 % MeOH) 0,30 | 83 % |
| 5'-O-(4,4'-DiNppoc-trity)I-thymidin | 4+1 h | (EE/Hex 3:1) 0,34 | 26 % |
| 5'-O-(4-Methoxy-4',4"-diNppoc-trityl)-thymidin | 4+1 h | (EE/Hex 2:1) 0,21 | 44 % |
| 5'-O-(4,4'4"-TriNppoc-trity)l-thymidin | 4+ 1 h | (EE/Hex 3:1) 0,25 | 13 % |

Man erhält das 5'-Triphenylmethyl-Thymidin Derivate als weißen festen Schaum in Ausbeuten zwischen 13-95 % d. Th.

### NMR-Daten zu 5'-O-(4-Methoxy-4'-(2-[2-nitrophenyl]-propyloxycarbonyloxy)-trityl)-thymidin

¹³C-NMR: (125.75 MHz, CDCl₃, TMS): δ=11.78 (5-CH3), 17.60 (CH3-nppoc), 33.10 (CH-nppoc), 40.72 (C-2'), 55.15 (O-CH3), 63.67 (C-5'), 70.95 (C-3'), 72.11 (CH2-nppoc), 84.65 (C-4'), 85.03 (C-1 '), 86.64 (C-trityl), 111.20 (C-5), 113.25 (C-3,5-methoxyphenyl(trityl)), 120.35 (C-3,5-nppocphenyl(trityl)), 124.27 (C-3-phenyl-nppoc), 127.27 (C-4-phenyl(trityl)), 127.69 (C-6-phenyl-nppoc), 127.79 (C-2,6-phenyl (trityl)), 128.19(C-4-phenyl-nppoc), 128.98 (C-4-phenyl-nppoc), 129.07 (C-2,6-nppocphenyl(trityl)), 134.35 (C-1-methoxyphenyl(trityl)), 135.54 (C-6), 136.52 (C-1-phenyl-nppoc), 141.60 (C-1-nppocphenyl(trityl)), 144.91 (C-1-phenyl(trityl)), 149.80 (C-2-phenyl-nppoc), 150.08 (O(CO)O), 150.61 (C-2), 153.31 (C-4-nppocphenyl(trityl)), 158.74 (C-4-methoxyphenyl(trityl)), 164.17 ppm (C-4).

### 1.14 4-Methoxy-4'-(2-[2-nitrophenyl]-propyloxycarbonyloxy)-triphenylcarbinol (S2)

Zur schutzgruppenfreie Synthese wurden in einem ausgeheizten Kolben 0,50 g (20,60 mmol, 1,2 eq) Magnesium mit 20 ml THF unter Argon suspendiert. 3,2 ml (25,70 mmol, 1,5 eq) Bromanisol wurden mit einem Tropftrichter langsam unter konstantem Sieden zugetropft. Nach Beendigung der Zugabe wurde mit einem Ölbad auf 85 °C erhitzt und 15 Minuten lang gerührt. Das entstandene Magnesiumbromid wurde auf 70 °C abkühlen gelassen und 3,40 g (17,17 mmol, 1 eq) 4-Hydroxybenzophenon, gelöst in 50 ml THF, zugetropft und für weitere 2 h bei 70 °C gerührt. Anschließend wurde der Ansatz erkalten gelassen und mit gesättigter Amoniumchlorid- Lösung die Reaktion abgebrochen. Zum Extrahieren wurden 250 ml Ethylacetat zugegeben und 2x mit Amoniumchlorid-Lösung (sat.) und 1x mit Natriumchlorid-Lösung (sat.) extrahiert. Die organische Phase wurde mit Natriumsulfat getrocknet und eingedampft. Auf eine Aufarbeitung wurde verzichtet und mit Pyridin mehrfach coevaporiert.

Zu dem in 40 ml Pyridin/Methylenchlorid (1:1) gelösten Ansatz wurden 11,00 g (45,00 mmol) Nppoc-Cl gelöst, in 15ml Methylenchlorid, zugetropft und 16 h bei RT gerührt. Danach wurde eingeengt, in Methylenchlorid gelöst, 2x mit Natriumhydrogencarbonat-Lösung (1 N) und 1x mit Natriumchlorid-Lösung (sat.) extrahiert. Die organische Phase wurde getrocknet und eingedampft. Der Rückstand wurde säulenchromatographisch nach dem Stufengradientenverfahren an Kieselgel 40-63 µm, Eluent Ethylacetat/n-Hexan + 1 % TEA (1:3 und 1:2), gereinigt. Man erhält das 4-Methoxy-4'-Nppoc-triphenylcarbinol als weißen festen Schaum.

Ausbeute: 3,7 g ≈ 42 % d. Th.

R_{f}-Wert _{(Ethylacetat / n-Hexan 1:1 + 1 % TEA)}: 0,54

### 1.15 5'-O-(4,4',4"-Tri-pivaloyl-triphenylmethyl)-Thymidin aus Rosolsäure

Zu 5 g (17,22 mmol) p-Rosolsäure, gelöst in 75 ml Pyridin und 2,5 ml 2,6-Lutidin, wurden 10 ml (9,8 mmol, 4,7 eq) Pivaloylsäurechlorid zugetropft. Anschließend wurde bei 70 °C für 80 min gerührt. Beim Erkalten lassen bildeten sich Kristalle, die isoliert und mit Pyridin gespült wurden. 1 g (1,73 mmol) der Kristalle wurden in 25 ml Methylenchlorid gelöst, unter DMAP-Katalyse (20 mg, 0,17 mmol), mit 20 ml 2,6-Lutidin und 220 mg (0,86 mmol) Thymidin umgesetzt. Danach wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch nach dem Stufengradientenverfahren an Kieselgel 40-63 µm, Eluent Ethylacetat/n-Hexan + 1 % TEA (1:3, 1:2), gereinigt. Man erhält das 5'-O-(4,4',4"-Tri-pivaloyl-triphenylmethyl)-Thymidin als weißen festen Schaum.

Ausbeute: 0,18 g ≈ 13,15 % d. Th.

¹³C-NMR: (125.75 MHz, CDCl₃, TMS): δ=12.11 (5-CH3), 27.02 (CH3-piv), 38.93 (C-piv), 41.02 (C-2'), 63.92 (C-5'), 70.33 (C-3'), 84.53 (C-4'), 85.92 (C-1'), 86.20 (C-trityl), 110.82 (C-5), 120.97 (C-3,3',3", 5,5',5"-trityl), 129.59 (C-2,2',2",6,6',6"-trityl), 135.09 (C-6), 140.47 (C-1,1',1"-trityl), 151.00 (C-2), 154.21 (C-4,4',4"-trityl), 164.10 (C-4), 176.78 ppm (C=O-piv).

### 1.16 5'-O-(4,4",4"-Tri-(2-[2-nitrophenyl]-propyloxycarbonyloxy)-triphenylmethyl)-deoxythymidin aus Rosolsäure

10 g (34,44 mmol) p-Rosolsäure, gelöst in 120 ml Pyridin und 30 ml 2,6-Lutidin, wurde auf 50 °C erwärmt und 44 g (178,28 mmol) Nppoc-Cl zugetropft. Nach 16 h Reaktionszeit bei 50 °C wurde überschüssiges Chlorid durch Zugabe von t-Buthanol gequencht. Die Umsetzung wurde mittels DC überprüft (R_{f}-Wert _{(Ethylacetat / n-Hexan 1:1 +1 % TEA)}: 0,35). Nach weiteren 30 min Reaktionszeit wurden bei einer Temperatur von 45 °C 8,2 g Thymidin und 750 mg DMAP zugegeben. Nach 16 h Rühren wurde eingeengt, in Methylenchlorid gelöst, 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch an Kieselgel 40-63 µm, Eluent Ethylacetat/n-Hexan + 1 % TEA (3:1), gereinigt. Man erhält das 5'-O-(4,4',4"-Tri-(2-[2-nitrophenyl]-propyloxycarbonyloxy)-triphenylmethyl)-deoxythymidin als weißen festen Schaum.

Ausbeute: 10 g ≈ 25 % d. Th.

R_{f}-Wert _{(Ethylacetat / n-Hexan 3:1 +1 % TEA)}: 0, 25
¹³C-NMR: (125.75 MHz, CDCl₃, TMS): δ=12.00 (5-CH3), 17.59 (CH3-nppoc), 33.06 (CH-nppoc), 40.50 (C-2'), 63.74 (C-5'), 71.48 (C-3'), 72.14 (CH2-nppoc), 84.55 (C-4'), 85.52 (C-1'), 86.16 (C-trityl), 111.13 (C-5), 120.58 (C-3,3',3",5,5',5"-trityl), 124.26 (C-3-phenyl-nppoc), 127.63 (C-6-phenyl-nppoc), 128.19 (C-4-phenyl-nppoc), 129.58 (C-2,2',2",6,6',6"-trityl), 135.29 (C-6), 136.47 (C-1-phenyl-nppoc), 140.53(C-1,1',1"-trityl), 150.09 (C-2-phenyl-nppoc), 150.34,(O(CO)O), 150.34 (C-2), 153.13 (C-4,4',4"-trityl), 163.73 ppm (C-4).
MS (TOF, ES⁺): 1175,89 [M +Na], 1191,83[M+K].
MS (TOF, ES⁻): 1152,87 [M-H].
C_{59H}55N₅O₂₀: 1154,12

### 1.17 5'-O-(4,4',4"-Tri-(2-[2-nitrophenyl]-propyloxycarbonyloxy)-triphenylmethyl)-N4-isobutryl-deoxycytidin

2,8 g (9,64 mmol) p-Rosolsäure, gelöst in 20 ml Pyridin und 20 ml 2,6-Lutidin, wurden auf 50 °C erwärmt und 14,0 g (56,72 mmol) Nppoc-Cl zugetropft. Nach 30 min wurde überschüssiges Chlorid durch Zugabe von t-Butanol gequencht. Die Umsetzung wurde mittels DC überprüft (R_{f}-Wert _{(Methylenchlorid/Methanol 5 % +1 % TEA)}: 0,36). Nach weiteren 30 min Reaktionszeit bei einer Temperatur von 45 ° C wurden 3,80 g N⁴-Isobutryl-deoxycytidin und 250 mg DMAP, gelöst in 30ml DMF, zugegeben. Nach 36 h wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch an Kieselgel 40-63 µm, Eluent Ethylacetat/n-Hexan + 1 % TEA (3:1), gereinigt. Man erhält das 5'-O-(4,4',4"-Tri-(2-[2-nitrophenyl]-propyloxycarbonyloxy)-triphenylmethyl)-N⁴-isobutryl-deoxycytidin als weißen festen Schaum.

Ausbeute: 2,6 g ≈ 16,8 % d. Th.

R_{f}-Wert _{(Ethylacetat / n-Hexan 3:1 +1 % TEA)}: 0,25
¹³C-NMR: (125.75 MHz, CDCl₃, TMS): δ=17.61 (CH3-nppoc), 18.89 (CH3-ibu), 33.10 (CH-nppoc), 36.10 (CH-ibu), 41.69 (C-2'), 63.39 (C-5'), 70.67 (C-3'), 72.10 (CH2-nppoc), 85.90 (C-4'), 86.18 (C-trityl), 87.06 (C-1'), 96.45 (C-5), 120.60 (C-3,3',3", 5,5',5"-trityl), 124.27 (C-3-phenyl-nppoc), 127.62 (C-6-phenyl-nppoc), 128.21 (C-4-phenyl-nppoc), 129.55 (C-2,2',2",6,6',6"-trityl), 132.72 (C-5-phenyl-nppoc), 136.50 (C-1-phenyl-nppoc), 140.50 (C-1,1',1"-trityl), 143.85 (C-6), 150.08 (O(CO)O), 150.08 (C-2-phenyl-nppoc), 153.15 (C-4,4',4"-trityl), 155.32 (C-2), 162.32 (C-4), 176.91 ppm (C=O-ibu).
MS (TOF, ES⁺): 1209,48 [M + H], 1231,48 [M+Na].
MS (TOF, ES.): 1209,48 [M-H].
C₆₂H₆₀N₆H₂₀: 1209,20

### 1.18 5'-O-(4,4',4"-Tri-(2-[2-nitrophenyl]-propyloxycarbonyloxy)-triphenylmethyl)-N⁶-pivaloyl-deoxyadenosin

2,8 g (9,64 mmol) p-Rosolsäure, gelöst in 20 ml Pyridin und 20 ml 2,6-Lutidin, wurden auf 50 °C erwärmt und 14,0 g (56,72 mmol) Nppoc-Cl zugetropft. Nach 30 min wurde überschüssiges Chlorid durch Zugabe von t-Butanol gequencht. Die Umsetzung wurde mittels DC überprüft (R_{f}-Wert _{(Methylenchlorid/Methanol 5% +1 % TEA)}: 0,36). Nach weiteren 30 min Reaktionszeit bei einer Temperatur von 45 ° C wurden 5,7 g N⁶-Pivaloyl-deoxyadenosin und 250 mg DMAP, gelöst in 30 ml DMF, zugegeben. Nach 36 h wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch an Kieselgel 40-63 µm, Eluent Ethylacetat/n-Hexan + 1 % TEA (3:1), gereinigt. Man erhält das 5'-O-(4,4',4"-Tri-(2-[2-nitrophenyl]-propyloxycarbonyloxy)-triphenylmethyl)-N⁶-pivaloyl-deoxyadenosin als weißen festen Schaum.

Ausbeute: 1,3 g ≈ 6,8 % d. Th.

¹³C-NMR: (125.75 MHz, CDCl₃, TMS): δ=17.56 (CH3-nppoc), 27.40 (CH3-piv), 33.04 (CH-nppoc), 39.69 (C-2'), 40.32 (C-piv), 63.73 (C-5'), 71.48 (C-3'), 72.35 (CH2-nppoc), 84.45 (C-4'), 85.82 (C-trityl), 85.90 (C-1'), 120.39 (C-3,3',3",5,5',5"-trityl), 122.61 (C-5), 124.21 (C-3-phenyl-nppoc), 127.58 (C-6-phenyl-nppoc), 128.17 (C-4-phenyl-nppoc), 129.51 (C-2,2',2",6,6',6"'-trityl), 132.59 (C-5-phenyl-nppoc), 136.67 (C-1-phenyl-nppoc), 140.68 (C-1,1',1"-trityl), 141.32 (C-8), 149.90 (C-4), 149.93 (C-2-phenyl-nppoc), 150.03 (O(CO)O), 151,02 (C-6), 152.30 (C-2), 153.10 (C-4,4',4"-trityl), 175.67 ppm (C=O-piv).
MS (TOF, ES⁺): 1247,49 [M+H], 1270,48 [M+Na].
MS (TOF, ES⁻): 1245,46 [M-H].
C₆₄H₆₃N₈O₁₉: 1247,25

### 1.19 5'-O-(4,4',4"-Tri-(2-[2-nitrophenyl]-propyloxycarbonyloxy)-triphenylmethyl)-N2-isobutryl-deoxyguanosin

4,0 g (13,8 mmol) p-Rosolsäure, gelöst in 50 ml Pyridin und 30 ml 2,6-Lutidin, wurden auf 50 °C erwärmt und 18,0 g (73,9 mmol) Nppoc-Cl zugetropft. Nach 16 h wurde überschüssiges Chlorid durch Zugabe von t-Butanol gequencht. Die Umsetzung wurde mittels DC überprüft (R_{f}-Wert _{(Ethylacetat / n-Hexan 1:1 +1 % TEA)}: 0,36). Nach weiteren 30 min Reaktionszeit bei einer Temperatur von 40 °C wurden 4,60 g N²-Isobutryl-deoxyguanosin und 250 mg DMAP, gelöst in 30 ml DMF, zugegeben. Nach 36 h wurde eingeengt, in Methylenchlorid gelöst und 2x mit Natriumhydrogencarbonat-Lösung (1N) und 1x mit Natriumchlorid- Lösung (sat.) extrahiert. Die organische Phase wurde getrocknet und eingedampft. Der Rückstand wurde säulenchromatographisch an Kieselgel 40-63 µm, Eluent Ethylacetat/n-Hexan + 1 % TEA (3:1), gereinigt. Man erhält das 5 '-O-(4,4',4' -Tri-(2-[2-nitrophenyl]-propyloxycarbonyloxy)-triphenylmethyl)-N²-isobutryl-deoxyguanosin als weißen festen Schaum. (Methylenchlorid/Methanol 5% + 1% TEA): 0,22

Ausbeute: 1,3 g ≈ 7,0 % d. Th.

¹³C-NMR: (125.75 MHz, CDCl₃, TMS): δ=17.63 (CH3-nppoc), 18.83 (CH3-ibu), 33.12 (CH-nppoc), 35.90 (CH-ibu), 39.89 (C-2'), 64.19 (C-5'), 70.97 (C-3'), 72.10 (CH2-nppoc), 83.90 (C-4'), 85.66 (C-trityl), 85.82 (C-1'), 120.31 (C-5), 120.34 (C-3,3',3",5,5",5-trityl), 124.21 (C-3-phenyl-nppoc), 127.61 (C-6-phenyl-nppoc), 128.25 (C-4-phenyl-nppoc), 129.57 (C-2,2',2",6,6',6"-trityl), 132.78 (C-5-phenyl-nppoc), 136.47 (C-1-phenyl-nppoc), 137.74 (C-8), 140.82 (C-1,1',1"-trityl), 147.91 (C-4), 148.37 (C-2), 149.92 (C-2-phenyl-nppoc), 150.04 (O(CO)O), 153.19 (C-4,4',4"-trityl), 155.94 (C-6), 179.84 ppm (C=O-ibu). C₆₃H₆₀N₈O₂₀: 1249,22

### 1.20 5'-O-(4,4',4"-Tri-(2-[2-nitrophenyl]-propyloxycarbonyloxy)-triphenylmethyl)-N⁶-pivaloyl-deoxyadenosin-β-cyanoethyl-N,N-diisopropylaminophosphoamidit

1,0 g (0,80 mmol) des 5'-Triphenylmethyldeoxyadenosin-Derivates wurden in Methylenchlorid und 2,6 eq Ethyldiisopropylamin gelöst und unter Schutzgas auf 0 °C abgekühlt. Anschließend wurden 1,2 eq Chlorphosphin zugetropft und 10 min bei 0 °C gerührt. Es wurde 1 h bei RT gerührt und mit Ethanol die Reaktion abgebrochen. Danach wurde eingeengt und in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch mit Methylenchlorid/Methanol 2 % an Kieselgel 40-63 µm gereinigt. Man erhält das 5'-O-(4,4',4"'-Tri-(2-[2-nitrophenyl]-propyloxycarbonyloxy)-triphenylmethyl)-N'-pivaloyl-deoxyadenosin-β-cyanoethyl-N,N-diisopropylaminophosphoamidit als weißen festen Schaum.

Ausbeute: 0,7 g ≈ 60 % d. Th.

³¹P-NMR (202,45 MHz, CDCl₃): δ=149,37 ppm.

### 1.21 5'-O-(4,4',4"-Tri-(2-[2-nitrophenyl]-propyloxycarbonyloxy)-triphenylmethyl)-thymidin-β-cyanoethyl-N,N-diisopropylaminophosphoamidit

2,0 g (1,73 mmol) des 5'-Triphenylmethylthymidin-Derivates wurden in Methylenchlorid und 2,6 eq Ethyldiisopropylamin gelöst und unter Schutzgas auf 0 °C abgekühlt. Anschließend wurden 1,2 eq Chlorphosphin zugetropft und 10 Minuten bei 0 °C gerührt. Es wurde 1 h bei RT gerührt und mit Ethanol die Reaktion abgebrochen. Danach wurde eingeengt, in Methylenchlorid gelöst und 2x mit Natriumhydrogencarbonat-Lösung (1N) und 1x mit Natriumchlorid-Lösung (sat.) extrahiert. Die organische Phase wurde getrocknet und eingedampft. Der Rückstand wurde säulenchromatographisch mit Essigsäureethylester/Hexan 1: an Kieselgel 40-63 µm gereinigt. Man erhält das 5'-O-(4,4',4"-Tri-(2-[2-nitrophenyl]-propyloxycarbonyloxy)-triphenylmethyl)-thymidin-β-cyanoethyl-N,N-diisopropylaminophosphoamidit als weißen festen Schaum.

Ausbeute: 2,1 g ≈ 89 % d. Th.

³¹P-NMR (202,45 MHz, CDCl3): δ=149,77 ppm.

### Beispiel 2: Abspaltung von Schutzgruppen

Die Säurebeständigkeit der nicht aktivierten Schutzgruppen wurde mit 80 %iger Essigsäure bzw. 1 %iger Trichloressigsäure (Standardbedingungen für die Oligonukleotidsynthese) durchgeführt. Tabelle 2 gibt das Ergebnis dieser Studie wieder.

Alle Proben werden zunächst für 300 s bzw. 900 s in 80 %iger Essigsäure gelöst und auf der HPLC analysiert. Diejenigen, die diese Behandlung ohne Etherspaltung überstehen, werden für 300 s bzw. 900 s mit 1 %iger Trichloressigsäure behandelt und anschließend analysiert.

**Tabelle 2:**

| Verbindung | Symbol | 80 %ige Essigsäure | 1 %ige Trichloressigsäure |
|---|---|---|---|
| 5'-O-(4-Nppoc-trityl)-thymidin | S1 | stabil | labil |
| 5'-O-(4-Methoxy-4'-Nppoc-trityl)-thymidin | S2 | stabil | labil |
| 5'-O-(4,4'-Dimethoxy-4"-Nppoc-trityl)-thymidin | S3 | labil | labil |
| 5'-O-(4,4'-DiNppoc-trityl)-thymidin | S4 | stabil | labil |
| 5'-O-(4-Methoxy-4,4"-diNppoc-trityl)-thymidin | S5 | stabil | labil |
| 5'-O-(4,4',4"-TriNppoc-trityl)-thymidin | S6 | stabil | stabil |

Es zeigt sich, dass die 4,4',4"-TriNppoc-trityl-Schutzgruppe (S6) in Trichloressigsäure stabil ist.

### Beispiel 3: Optimierung der Synthese

Eine erste Optimierung wird durch Verwendung von Diphenylmethylchlorsilan anstelle von tert.Butoxydiphenylchlorsilan erzielt.

### Beispiel 4: 2 Experimente an der festen Phase

Die zweistufigen Schutzgruppen werden auf einer festen Phase getestet. Der prinzipielle Funktionalitätstest wird auf einem DNA-Prozessor D der febit ag in einem Geniom^{®} one-Gerät durchgeführt.

An eine silanisierte DNA-Prozessoroberfläche wird 5'-O-(4,4',4"-TriNppoc-trityl)-thymidin-phosphoamidit ankondensiert. Es erfolgt eine punktuelle Belichtung, eine anschließende Säurebehandlung (max. 2 min) und die Kopplung eines Spacers. Abschließend erfolgt nochmals eine Detektion.

Als Kontrolle erfolgt das analoge Experiment. Es wird jedoch statt des Amidits mit der Tandem-Schutzgruppe ein NPPOC-dT-Amidit gekoppelt. Durch dieses Experiment konnte die Funktionalität der zweistufigen Schutzgruppe gezeigt werden.

## Patentansprüche

1. Verfahren zur Synthese von Biopolymeren durch schrittweisen Aufbau aus Synthesebausteinen, die Schutzgruppen tragen,
**dadurch gekennzeichnet,**
**dass** man mindestens einen Synthesebaustein verwendet, der eine zweistufige Schutzgruppe trägt, wobei die zweistufige Schutzgruppe durch einen Belichtungsschritt aktiviert und einen anschließenden chemischen Behandlungsschritt abgespalten wird, und wobei man als zweistufige Schutzgruppe eine derivatisierte Tritylgruppe verwendet.

2. Verfahren nach Anspruch 1,
**dadurch gekennzeichnet,**
**dass** der chemische Behandlungsschritt eine Behandlung mit Base, eine Behandlung mit Säure, eine Oxidation, eine Reduktion oder/und eine enzymatische Reaktion umfasst.

3. Verfahren nach Anspruch 2,
**dadurch gekennzeichnet,**
**dass** der chemische Behandlungsschritt eine Säurebehandlung umfasst.

4. Verfahren nach einem der Ansprüche 1 bis 3,
**dadurch gekennzeichnet,**
**dass** der Synthesebaustein mit der zweistufigen Schutzgruppe die allgemeine Formel I aufweist: 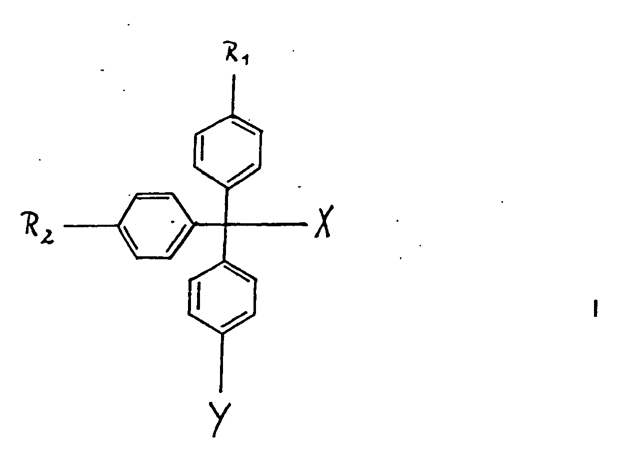 wobei R₁ und R₂ jeweils unabhängig ausgewählt sind aus Wasserstoff, OR₃, O(CH₂)ₙCOOR₃ und NHZ,
R₃ eine C₁-C₈ Alkylgruppe, eine C₂-C₈-Alkenylgruppe, eine C₂-C₈-Alkinylgruppe oder/und eine C₆-C₂₀ Arylgruppe enthält, die gegebenenfalls Substituenten aufweisen kann,
X den Synthesebaustein darstellt,
Y jeweils unabhängig eine fotoaktivierbare Schutzgruppe ist,
Z eine Aminoschutzgruppe ist,
n eine ganze Zahl von 0 bis 4 ist und
wobei gegebenenfalls R₁ oder/und R₂ durch Y ersetzt sein können.

5. Verfahren nach Anspruch 4,
**dadurch gekennzeichnet,**
**dass** man eine zweistufige Schutzgruppe verwendet, die mindestens eine fluoreszierende Gruppe trägt.

6. Verfahren nach Anspruch 5,
**dadurch gekennzeichnet,**
**dass** Y, R₃ oder/und Z die fluoreszierende Gruppe tragen.

7. Verfahren nach Anspruch 5 oder 6,
**dadurch gekennzeichnet,**
**dass** die fluoreszierende Gruppe durch Substitution am Trityl-Gerüst eingeführt wird.

8. Verfahren nach einem der Ansprüche 1 bis 7,
**dadurch gekennzeichnet,**
**dass** die fotoaktivierbare Gruppe der zweistufigen Schutzgruppe ausgewählt wird aus Nitroveratryloxycarbonyl (NVOC), α-Methyl-6-nitropiperonyloxycarbonyl (MeNPOC), 3,5-Dimethoxybenzoin-carbonat (DMBOC), 2-(o-Nitrophenyl)propyloxycarbonyl (NPPOC), o-Nitrobenzyl und Derivaten davon und 2-(o-Nitrophenyl)ethyl und Derivaten davon.

9. Verfahren nach einem der Ansprüche 1 bis 8,
**dadurch gekennzeichnet,**
**dass** die Biopolymere ausgewählt werden aus Nukleinsäuren, Nukleinsäureanaloga, Peptiden und Sacchariden.

10. Verfahren nach Anspruch 9,
**dadurch gekennzeichnet,**
**dass** die Biopolymere ausgewählt werden aus Nukleinsäuren und Nukleinsäureanaloga.

11. Verfahren nach Anspruch 10,
**dadurch gekennzeichnet,**
**dass** als Synthesebausteine Phosphoramidite verwendet werden.

12. Verfahren nach Anspruch 11,
**dadurch gekennzeichnet,**
**dass** Phosphoramiditbausteine verwendet werden, die die zweistufige Schutzgruppe am 5'-O-Atom tragen.

13. Verfahren nach einem der Ansprüche 1 bis 12,
**dadurch gekennzeichnet,**
**dass** die Synthese der Biopolymeren die Verwendung von Spacer- bzw. Linkerbausteinen umfasst.

14. Verfahren nach einem der Ansprüche 1 bis 13,
**dadurch gekennzeichnet,**
**dass** die Synthese der Biopolymeren auf einer Festphase durchgeführt wird.

15. Verfahren nach Anspruch 14,
**dadurch gekennzeichnet,**
**dass** eine ortsabhängige Synthese von mehreren Biopolymeren mit jeweils einer unterschiedlichen Sequenz von Synthesebausteinen auf einem einzigen Träger durchgeführt wird.

16. Verbindungen der allgemeinen Formel I 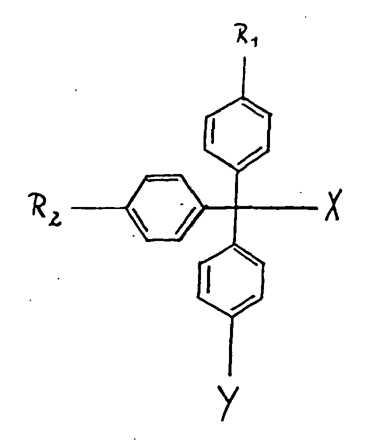 wobei R₁ und R₂ jeweils unabhängig ausgewählt sind aus Wasserstoff, OR₃, O(CH₂)ₙCOOR₃ und NHZ,
R₃ eine C₁-C₈ Alkylgruppe, eine C₂-C₈-Alkenylgruppe, eine C₂-C₈-Alkinylgruppe oder/und eine C₆-C₂₀ Arylgruppe enthält, die gegebenenfalls Substituenten aufweisen kann,
X einen Synthesebaustein zur Synthese von Biopolymeren oder eine Abgangsgruppe darstellt,
Y jeweils unabhängig eine fotoaktivierbare Schutzgruppe ist,
Z eine Aminoschutzgruppe ist,
n eine ganze Zahl von 0 bis 4 ist und
wobei gegebenenfalls R₁ oder/und R₂ durch Y ersetzt sein können.

17. Verbindungen nach Anspruch 16,
**dadurch gekennzeichnet,**
**dass** sie mindestens eine fluoreszierende Gruppe tragen.

18. Verbindungen nach Anspruch 17,
**dadurch gekennzeichnet,**
**dass** Y, R₃ oder/und Z eine fluoreszierende Gruppe tragen.

19. Verbindungen nach Anspruch 17 oder 18,
**dadurch gekennzeichnet,**
**dass** eine fluoreszierende Gruppe durch Substitution am Trityl-Grundgerüst eingeführt ist.

20. Verwendung von Verbindungen der allgemeinen Formel I als Synthesebausteine bzw. zur Herstellung von Synthesebausteinen für die Synthese von Biopolymeren.

## Claims

1. Process for the synthesis of biopolymers by stepwise assembly from synthesis building blocks which carry protective groups,
**characterized in that**
at least one synthesis building block which carries a two-stage protective group is used, where the two-stage protective group is activated by an illumination step and is cleaved off by a subsequent chemical treatment step, and a derivatized trityl group is used as the two-stage protective group.

2. Process according to claim 1,
**characterized in that**
the chemical treatment step comprises a treatment with a base, a treatment with an acid, an oxidation, a reduction or/and an enzymatic reaction.

3. Process according to claim 2,
**characterized in that**
the chemical treatment step comprises an acid treatment.

4. Process according to one of the claims 1 to 3,
**characterized in that**
the synthesis building block with the two-stage protective group has the general formula I: 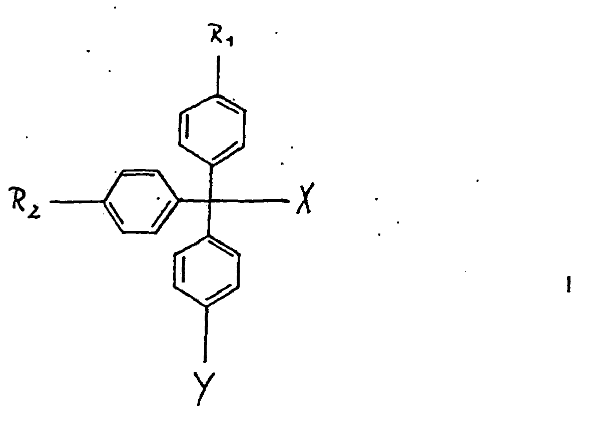 in which R₁ and R₂ are each independently selected from hydrogen, OR₃, O(CH₂)ₙCOOR₃ and NHZ,
R₃ comprises a C₁- C₈ alkyl group, a C₂ - C₈ alkenyl group, a C₂ - C₈ alkynyl group or/and a C₆ - C₂₀ aryl group which may optionally have substituents,
X represents the synthesis building block,
Y is in each case independently a photoactivatable protective group,
Z is an amino protective group,
n is an integer from 0 to 4 and
in which R₁ or/and R₂ may optionally be replaced by Y.

5. Process according to claim 4,
**characterized in that**
a two-stage protective group which carries at least one fluorescent group is used.

6. Process according to claim 5,
**characterized in that**
Y, R₃ or/and Z carry the fluorescent group.

7. Process according to claim 5 or 6,
**characterized in that**
the fluorescent group is introduced by substitution on the trityl framework.

8. Process according to one of the claims 1 to 7,
**characterized in that**
the photoactivatable group of the two-stage protective group is selected from nitroveratryloxycarbonyl (NVOC), α-methyl-6-nitropiperonyloxycarbonyl (MeNPOC), 3,5-dimethoxybenzoin carbonate (DMBOC), 2-(o-nitrophenyl)-propyloxycarbonyl (NPPOC), o-nitrobenzyl and derivatives thereof and 2-(o-nitrophenyl)ethyl and derivatives thereof.

9. Process according to one of the claims 1 to 8,
**characterized in that**
the biopolymers are selected from nucleic acids, nucleic acid analogues, peptides and saccharides.

10. Process according to claim 9,
**characterized in that**
the biopolymers are selected from nucleic acids and nucleic acid analogues.

11. Process according to claim 10,
**characterized in that**
phosphoramidites are used as synthesis building blocks.

12. Process according to claim 11,
**characterized in that**
phosphoramidite building blocks which carry the two-stage protective group on the 5'-O-atom are used.

13. Process according to one of the claims 1 to 12,
**characterized in that**
the synthesis of the biopolymers comprises the use of spacer or linker building blocks.

14. Process according to one of the claims 1 to 13,
**characterized in that**
the synthesis of the biopolymers is carried out on a solid phase.

15. Process according to claim 14,
**characterized in that**
a site-dependent synthesis of a plurality of biopolymers each with a different sequence of synthesis building blocks is carried out on a single support.

16. Compounds of the general formula I 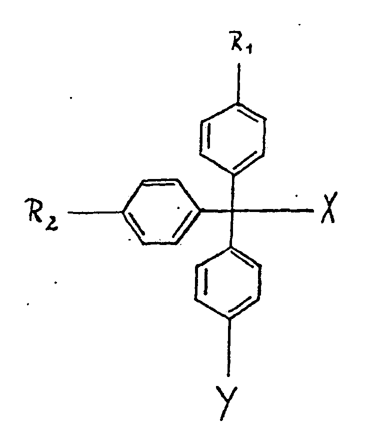 in which R₁ and R₂ are each independently selected from hydrogen, OR₃, O(CH₂)ₙCOOR₃ and NHZ,
R₃ comprises a C₁ - C₈ alkyl group, a C₂ - C₈ alkenyl group, a C₂ - C₈ alkynyl group or/and a C₆ - C₂₀ aryl group which may optionally have substituents,
X is a synthesis building block for the synthesis of biopolymers or a leaving group,
Y is in each case independently a photoactivatable protective group,
Z is an amino protective group,
n is an integer from 0 to 4 and
in which R₁ or/and R₂ may optionally be replaced by Y.

17. Compounds according to claim 16,
**characterized in that**
they carry at least one fluorescent group.

18. Compounds according to claim 17,
**characterized in that**
Y, R₃ or/and Z carry a fluorescent group.

19. Compounds according to claim 17 or 18,
**characterized in that**
a fluorescent group is introduced by substitution on the trityl framework.

20. Use of compounds of the general formula I as synthesis building blocks or for the preparation of synthesis building blocks for the synthesis of biopolymers.

## Revendications

1. Procédé pour la synthèse, par étapes, de biopolymères à partir de modules de synthèse qui portent des groupes protecteurs, **caractérisé en ce que** l'on utilise au moins un module de synthèse qui porte un groupe protecteur à deux étapes, le groupe protecteur à deux étapes étant activé par une étape d'exposition à la lumière puis éliminé par une étape de traitement chimique, le groupe protecteur à deux étapes utilisé étant un groupe trityle.

2. Procédé selon la revendication 1, **caractérisé en ce que** l'étape de traitement chimique comporte un traitement avec une base, un traitement avec un acide, une oxydation, une réduction et/ou une réaction enzymatique.

3. Procédé selon la revendication 2, **caractérisé en ce que** l'étape de traitement chimique comprend un traitement avec un acide.

4. Procédé selon l'une quelconque des revendications 1 à 3, **caractérisé en ce que** le module de synthèse avec le groupe protecteur à deux étapes présente la formule générale 1 : 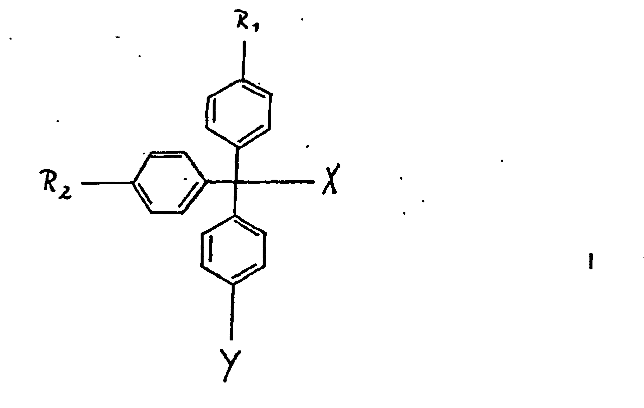 dans laquelle R₁ et R₂ sont choisis chacun indépendamment parmi un atome d'hydrogène, OR₃, O(CH₂)ₙCOOR₃ et NHZ,
R₃ contient un groupe alkyle en C₁ à C₈, un groupe alcényle en C₂ à C₈, un groupe alcynyle en C₂ à C₈ et/ou un groupe aryle en C₆ à C₂₀, qui peuvent comporter le cas échéant des substituants,
X représente le module de synthèse,
chaque Y est indépendamment un groupe de protection photoactivable,
Z est un groupe de protection amine,
n est un nombre entier de 0 à 4 et
dans laquelle R₁ ou/et R₂ peuvent être remplacés le cas échéant par Y.

5. Procédé selon la revendication 4, **caractérisé en ce que** l'on utilise un groupe protecteur à deux étapes qui porte au moins un groupe fluorescent.

6. Procédé selon la revendication 5, **caractérisé en ce que** Y, R₃ et/ou Z portent le groupe fluorescent.

7. Procédé selon la revendication 5 ou 6, **caractérisé en ce que** le groupe fluorescent est introduit par substitution sur le squelette trityle.

8. Procédé selon l'une quelconque des revendications 1 à 7, **caractérisé en ce que** le groupe photoactivable du groupe de protection à deux étapes est choisi parmi le nitrovératryloxycarbonyle (NVOC), le α-méthyl-6-nitropipéronyloxycarbonyle (MeNPOC), le 3,5-diméthoxybenzoïne-carbonate (DMBOC), le 2-(o-nitrophényl)propyloxycarbonyle (NPPOC), l'o-nitrobenzyle et des dérivés de celui-ci, et le 2-(o-nitrophényl)éthyle et des dérivés de celui-ci.

9. Procédé selon l'une quelconque des revendications 1 à 8, **caractérisé en ce que** les biopolymères sont choisis parmi les acides nucléiques, les analogues d'acides nucléiques, les peptides et les saccharides.

10. Procédé selon la revendication 9, **caractérisé en ce que** les biopolymères sont choisis parmi les acides nucléiques et les analogues d'acides nucléiques.

11. Procédé selon la revendication 10, **caractérisé en ce que** des phosphoramidites sont utilisés comme modules de synthèse.

12. Procédé selon la revendication 11, **caractérisé en ce que** l'on utilise des phosphoramidites qui portent le groupe protecteur à deux étapes sur l'atome d'oxygène de l'extrémité 5'.

13. Procédé selon l'une quelconque des revendications 1 à 12, **caractérisé en ce que** la synthèse des biopolymères comporte l'utilisation de modules espaceurs ou modules de liaison.

14. Procédé selon l'une quelconque des revendications 1 à 13, **caractérisé en ce que** la synthèse des biopolymères est effectuée sur une phase solide.

15. Procédé selon la revendication 14, **caractérisé en ce que** l'on met en oeuvre, sur un seul support, une synthèse localement différentielle de plusieurs biopolymères ayant chacun une séquence différente de modules de synthèse.

16. Composés de formule générale I 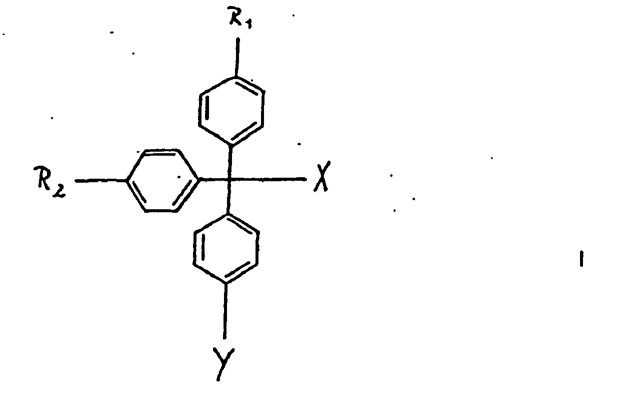 dans laquelle R₁ et R₂ sont choisis chacun indépendamment parmi un atome d'hydrogène, OR₃, O(CH₂)ₙCOOR₃ et NHZ,
R₃ contient un groupe alkyle en C₁ à C₈, un groupe alcényle en C₂ à C₈, un groupe alcynyle en C₂ à C₈ et/ou un groupe aryle en C₆ à C₂₀, qui peuvent comporter le cas échéant des substituants,
X représente un module de synthèse pour la synthèse de biopolymères ou un groupe partant,
chaque Y est indépendamment un groupe de protection photoactivable,
Z est un groupe de protection amine,
n est un nombre entier de 0 à 4 et
dans laquelle R₁ ou/et R₂ peuvent être remplacés le cas échéant par Y.

17. Composés selon la revendication 16, **caractérisé en ce qu'**ils portent au moins un groupe fluorescent.

18. Composés selon la revendication 17, **caractérisé en ce que** Y, R₃ ou/et Z portent un groupe fluorescent.

19. Composés selon la revendication 17 ou 18, **caractérisé en ce que** un groupe fluorescent est introduit par substitution sur le squelette de base trityle.

20. Utilisation de composés de la formule générale I comme modules de synthèse ou pour la préparation de modules de synthèse pour la synthèse de biopolymères.
